# EUROPEAN PATENT SPECIFICATION

(11) **EP 1 788 879 B1**
(45) Date of publication and mention of the grant of the patent: **23.12.2009**
(21) Application number: 05810024.9
(22) Date of filing: 07.09.2005
(51) Int. Cl.: A01N 37/02, A01N 37/36, A01N 65/00, A01N 37/16

(54) **ANTISEPTIC COMPOSITIONS AND METHODS OF USE**
ANTISEPTISCHE ZUSAMMENSETZUNGEN UND VERWENDUNGSVERFAHREN
COMPOSITIONS ANTISEPTIQUES ET LEURS METHODES D'UTILISATION

(30) Priority: 07.09.2004 US 936133
(43) Date of publication of application: 30.05.2007
(73) Proprietor: 3M Innovative Properties Company, St. Paul MN 55133-3427 (US)
(72) Inventor: SCHOLZ, Matthew T., 3M Center, Saint Paul, Minnesota 55133-3427 (US); HOBBS, Terry R.,, Saint Paul, Minnesota 55133-3427 (US)
(74) Representative: Vossius & Partner
(86) International application number: PCT/US2005/032228
(87) International publication number: WO 2006/029351

(56) References cited:
- WO-A-93/15018
- WO-A-99/59538
- DE-A1- 4 302 812
- BODDIE R L: "evaluation of postmilking teat germicides containing Lauricidin,saturated fatty acids , and lactic acid" STN CAPLUS, vol. 6, no. 117, 1992, XP002030991

## Description

The use of antimicrobial agents plays an important part in current medical therapy. This is particularly true in the fields of dermatology as well as skin and wound antisepsis, where the most effective course of treatment for skin or mucous membranes, which are afflicted with bacterial, fungal, or viral infections or lesions, frequently includes the use of a topical antimicrobial agent, such as antibiotics. For decades medicine has relied primarily upon antibiotics to fight systemic as well as topical infections.

Antibiotics are organic molecules produced by microorganisms that have the capacity in dilute solutions (e.g., solutions less than 10 µg/ml and often less than 1 µg/ml) to destroy or inhibit the growth of bacteria and other microorganisms. They are generally effective at very low levels and are often safe with very few, if any, side effects. Commonly, antibiotics may have a narrow spectrum of antimicrobial activity. Furthermore, they often act on very specific sites in cell membranes or on very specific metabolic pathways. This can tend to make it relatively easy for bacteria to develop resistance to the antibiotic(s) (i.e., the genetically acquired ability to tolerate much higher concentrations of antibiotic) either through natural selection, transmission of plasmids encoding resistance, mutation, or by other means. Not only does resistance eliminate the ability of a medication to treat an affliction, but it can also put the patient at further risk, especially if the antibiotic is one that is routinely used systemically.

In the past few decades it as been quite well established that colonization of the anterior nares with *Staphylococcus aureus* (SA) can lead to multiple problems. Medicine has relied primarily upon antibiotics for nasal decolonization. For example, bacitracin, neomycin sulfate, polymyxin B sulfate, gentamicin, framycetin-gramicidin, lysostaphin, methicillin, rifampin, tobramycin, nystatin, mupirocin, and combinations thereof, have been used with varying success for nasal decolonization.

For example, nasal colonization with SA in presurgical patients has resulted in higher infection rates and higher rates of other nosocomial infections such as catheter infections. Nasal colonization with SA in hemodialysis patients has resulted in a much higher incidence of blood stream infections. Furthermore, it has been well established that the anterior nares is the ecological niche for SA colonization and thus spread of methicillin resistant staphylococcus aureus (MRSA) in a hospital or other health care facilities in the event of an outbreak can be mitigated by decolonizing the anterior nares of patients and healthcare workers.

Mupirocin, marketed as the calcium salt in Bactroban Nasal by Glaxo Smith Kline, is the only antibiotic approved by the Food and Drug Administration for nasal decolonization use in the United States. For example, there are multiple reports of resistance to mupirocin when used as a nasal decolonizing agent. Resistance rates have been reported as high as 25% and even as high as 50% (see, for example, E. Perez-Roth et al., Diag. Micro. Infect. Dis., 43:123-128 (2002) and H. Watanabe et al., J. Clin. Micro., 39(10): 3775-3777 (2001)). Even though presurgical decolonization of the anterior nares using mupirocin has been shown to decrease the risk of surgical site infection by as much as 2 to 10 times (T. Perl et al., Ann. Pharmacother., 32:S7-S16 (1998)), the high resistance rates to this antibiotic make it unsuitable for routine use.

Antiseptics, on the other hand, are synthetic molecules that destroy or inhibit microorganisms and virus by inhibiting metabolic pathways or altering the cell envelope or both. They tend to have broader spectrum of antimicrobial activity and often act by nonspecific means such as disruption of cell membranes, oxidation of cellular components, denaturation of proteins, etc. This nonspecific activity makes it difficult for microorganisms to develop clinical resistance to antiseptics. For example, there are very few reports of clinical resistance to antiseptics such as iodine, lower alcohols (ethanol, propanol, etc.), chlorhexidine, quaternary amine surfactants, chlorinated phenols, and the like. Some of these compounds, however, need to be used at concentrations that often result in irritation or tissue damage, especially if applied repeatedly. Furthermore, unlike antibiotics, many antiseptics are not active in the presence of high levels of organic compounds. For example, formulations containing iodine or quaternary ammonium compounds have been reported to be inactivated by the presence of organic matter such as that in nasal or vaginal secretions, and perhaps even on skin.

Many antiseptic compounds are viewed as irritants. For example, compositions containing iodine and/or chlorhexidine have been reported to cause skin irritation. This is particularly true for sensitive mucosal tissues, such as the anterior nares, nasal and esophageal cavities, which can have a high level of microbial colonization in certain otherwise healthy individuals, as well as individuals with infectious diseases such as chronic sinusitis. Additionally, due to the irritating nature many of these compounds may be unsuitable for application to irritated or infected dermal tissue to treat skin conditions, such as lesions from impetigo and shingles.

Also, for certain applications, especially in the nose and mouth, it is particularly desirable for the compositions to have little or no color, little or no odor, and an acceptable taste. Many antiseptics have undesirable characteristics, such as iodine and iodophors, which have an orange to brown color and a definite objectionable odor at concentrations typically employed for antisepsis.

Chlorhexidine gluconate (in combination with neomycin sulfate) has been suggested for use in nasal decolonization with limited success. For example, Naseptin is an antibiotic emulsified cream comprising neomycin sulphate (3250 units/g) and chlorhexidine gluconate (0.1%) that in combination destroys bacteria. The product also contains arachis oil, cetostearyl alcohol/ethylene oxide concentrate, cetostearyl alcohol in a water base. The product must be used 4 times/day over 10 days to eradicate nasal carriage of staphylococci. In addition, US Patent No. 6,214,866 discloses the use of chlorhexidine in combination with the antibiotic mupirocin.

Povidone-iodine has also been suggested for use in nasal decolonization (R.L. Hill and M.W. Casewell, Journal of Hospital Infection, 2000, Vol. 45, 198-205). Betadine Cream (5wt% povidone iodine) has been found to kill methicillin resistant staphylococcus aureus in vitro in an enrichment culture technique. Addition of nasal secretions decreased the activity of the povidone-iodine by 80-90wt% by reaction of the free iodine with the organic load. Other drawbacks of 5wt% povidone-iodine for use in patients included: 1) a very dark brown color, 2) a low pH which can cause irritation, 3) a strong iodine odor.

The formulation of components can affect the performance and potential irritation of antimicrobial agents. For example, many conventional antimicrobial compositions are too low in viscosity and/or too hydrophilic in nature to maintain sufficient substantivity and persistence to provide sufficient antimicrobial activity on moist tissue, such as the anterior nares or open, exuding, or infected lesions. It has been reported that the presence of solvents can diminish the antimicrobial activity of many antiseptics. Furthermore, it has been reported that many surfactants can reduce the efficacy of antiseptics by sequestering the antiseptic in micelles. (H. B. Kostenbauer Chapter 44 in Disinfection, Sterilization, and Preservation, First addition, 1968, C. A. Lawrence and S.S. Block). Additionally, surfactants are often implicated in contributing to irritation.

Thus, there is still a need for effective antimicrobial compositions that develop little resistance and are well-tolerated when used on mammalian tissue and especially on moist mammalian tissue such as in the nasal passages, anterior nares, vagina, and wounds.

The present invention provides antimicrobial compositions for killing or inactivating microorganisms on mammalian tissue as set out in the appended claims. Such compositions are typically useful when applied topically, particularly to mucosal tissues (i.e., mucous membranes), although a wide variety of surfaces can be treated. They can provide effective reduction, prevention, or elimination of microbes, particularly bacteria, fungi, and viruses. Preferably, the microbes are of a relatively wide variety such that the compositions of the present invention have a broad spectrum of activity.

Compositions of the present invention provide effective topical antimicrobial activity and are accordingly useful in the local treatment and/or prevention of conditions that are caused, or aggravated by, microorganisms (including viruses, bacteria, fungi, mycoplasma, and protozoa) on various tissues such as skin, wounds, and/or mucous membranes.

Significantly, certain embodiments of the present invention have a very low potential for generating clinical microbial resistance. Thus, such compositions can be applied multiple times over one or more days to treat topical infections or to eradicate unwanted bacteria (such as nasal colonization of Staphylococcus aureus). Furthermore, compositions of the present invention can be used for multiple treatment regimens on the same patient without the fear of generating antimicrobial resistance. This can be particularly important for chronically ill patients who are, in need of decolonization of the anterior nares before hemodialysis, for example, or for antiseptic treatment of chronic wounds such as diabetic foot ulcers.

Also, preferred compositions of the present invention have a generally low irritation level for skin, skin lesions, and mucosal membranes (including the anterior nares, nasal cavities, and nasopharangyl cavity). Also, certain preferred compositions of the present invention are substantive (i.e., resist removal by fluids) for relatively long periods of time to ensure adequate efficacy.

Compositions of the present invention include an antiseptic selected from the group consisting of C6-C14 alkyl carboxylic acids, C6-C14 alkyl carboxylate ester carboxylic acids, C8-C22 mono- or polyunsaturated carboxylic acids, and antimicrobial natural oils.

Importantly, the compositions of the present invention are capable of destroying microorganisms on or in mammalian tissue. Therefore, the concentrations employed are generally greater than those that have been used to simply preserve certain topically applied compositions, i.e., prevent the growth of microorganism in topical compositions for purposes other than antisepsis. For example, the concentration may be at least 0.1wt%, preferably at least 0.2wt% and more preferably at least 0.5wt%. Commonly, the antiseptics may be employed at concentration of at least 1%, preferably at least 2% and often at least 3% by weight of the composition. All weight percents are based on the total weight of a "ready to use" or "as used" composition.

Depending on the application, many of these compounds at these concentrations can be irritating if delivered in simple aqueous or hydrophilic vehicle formulations. Therefore the compositions of the present invention incorporate a substantial amount of a lipophilic or hydrophobic phase. The hydrophobic phase is comprised of one or more water insoluble components. If delivered in a hydrophobic phase, the irritation can be significantly reduced. The incorporation of the hydrophobic phase may significantly reduce the irritation potential of the present compositions. The hydrophobic component is an organic compound that is liquid, gelatinous, semisolid, or solid at 23°C and has a solubility in water of less than 5 wt-% at 23°C. Preferred hydrophobic phase components have a solubility in water of less than 0.5% by weight and often less than 0.1% by weight at 23°C. In addition, the antiseptic is preferably present at a concentration approaching or preferably exceeding the solubility limit of the hydrophobic phase.

Importantly, the compositions also have sufficient viscosity to prevent inhalation into the lungs if used in the nose for applications such as nasal decolonization. The relatively high viscosity of the compositions of the present invention also minimizes migration that can be associated with other compositions thus reducing irritation and mess. Despite the presence of the hydrophobic phase many of the antiseptic containing compositions exhibit very effective and rapid antimicrobial activity.

In addition, antimicrobial compositions that include hydrophilic components such as polyols (e.g., glycerin and polyethylene glycols) that themselves have little or no antimicrobial activity can considerably enhance the antimicrobial activity of the compositions. Preferably, the hydrophilic component includes a glycol, a lower alcohol ether, a short chain ester, and combinations thereof, wherein the hydrophilic component is soluble in water in an amount of at least 20 wt-% at 23°C.

The compositions of the present invention are preferably free of antibiotics.

Preferably, the compositions also include a surfactant selected from the group of sulfonate, a sulfate, a phosphonate, a phosphate, amphoteric, a poloxamer, a cationic surfactant, or mixtures thereof. The compositions also include an enhancer component comprising an alpha-hydroxy acid, a beta-hydroxy acid, a chelating agent, a (C1-C4)alkyl carboxylic acid, a (C6-C12)aryl carboxylic acid, a (C6-C12)aralkyl carboxylic acid, a (C6-C16)alkaryl carboxylic acid, a phenolic compound, a (C1-C10)alkyl alcohol, an ether glycol, or combinations thereof.

The present invention also provides various methods of use of compositions of the present invention. In one embodiment, the present invention provides for the use of the compositions for preventing and/or treating an affliction caused, or aggravated by, a microorganism on mammalian tissue, such as skin and/or a mucous membrane. The use includes contacting the mammalian tissue with an antimicrobial composition of the present invention.

In one embodiment, the present invention provides the use of the compositions for decolonizing at least a portion of the nasal cavities, anterior nares, and/or nasopharynx of a subject of microorganisms. The use includes contacting the nasal cavities, anterior nares, and/or nasopharynx with an antimicrobial composition of the present invention in an amount effective to kill one or more microorganisms in or on tissue.

In one embodiment, the present invention provides for the use of the compositions for decolonizing at least a portion of the throat/esophagus of a subject of microorganisms. The use includes contacting the esophageal cavity with an antimicrobial composition of the present invention in an amount effective to kill one or more microorganism in or on the tissue in the throat.

In one embodiment, the present invention provides for the use of the compositions for decolonizing at least a portion of the throat/esophagus of a subject of microorganisms. The use includes contacting the oral and/or nasal cavity with an antimicrobial composition of the present invention in an amount effective to allow a sufficient quantity of the composition to pass down the throat to reduce or eliminate bacterial colonization in or on the tissue in the throat.

In one embodiment, the present invention provides for the use of the compositions for decolonizing at least a portion of the oral cavity of a subject of microorganisms. The use includes contacting the oral cavity with an antimicrobial composition of the present invention in an amount effective to kill one or more microorganisms in or on the soft tissue in the oral cavity.

In one embodiment, the present invention provides for the use of the compositions for treating a respiratory affliction (e.g., chronic sinusitis) in a subject. The use includes contacting at least a portion of the respiratory system (particularly the upper respiratory system including the nasal cavities, anterior nares, and/or nasopharynx) with an antimicrobial composition of the present invention in an amount effective to reduce or eliminate bacterial colonization in or on the soft tissue in the respiratory system.

In one embodiment, the present invention provides for the use of the compositions for treating impetigo on the skin of a subject. The use includes contacting the affected area with an antimicrobial composition of the present invention in an amount effective to reduce or eliminate clinical signs of infection.

The present invention relates to the compositions for killing or inactivating microorganisms. Herein, to "kill or inactivate" means to render the microorganism ineffective by killing them (e.g., bacteria and fungi) or otherwise rendering them inactive (e.g., viruses). The present invention provides the compositions for killing bacteria such as *Staphylococcus spp., Streptococcus spp., Escherichia spp., Enterococcus spp.* (including antibiotic resistant strains such as vancomycin resistant *Enterococcu),* and *Pseudamonas spp.* bacteria, and combinations thereof, and more particularly *Staphylococcus aureus* (including antibiotic resistant strains such as methicillin resistant *Staphylococcus aureus), Staphylococcus epidermidis, Escherichia coli (E. coli), Pseudomonas aeruginosa (Pseudomonas ae.),* and *Streptococcus pyogenes,* which often are on or in the skin or mucosal tissue of a subject. This includes contacting the microorganism with an antimicrobial composition of the present invention in an amount effective to kill one or more microorganisms (e.g., bacteria and fungi) or inactivate one or more microorganisms (e.g., viruses, particularly herpes virus).

For example, in one embodiment, the present invention relates to the compositions for killing or inactivating microorganisms in the nose or nasal cavity of a subject. This includes contacting the affected area with an antimicrobial composition of the present invention in an amount effective to kill one or more microorganisms on or in the tissue in the nose or nasal cavity.

The compositions of the present invention can also be used for providing residual antimicrobial efficacy on a surface that results from leaving a residue or imparting a condition to the surface (e.g., skin, in the anterior nares, mucosal tissue, wound, or medical device that comes in contact with such tissues, but particularly skin, mucosal tissue, and/or wound) that remains effective and provides significant antimicrobial activity. This is accomplished by providing compositions with relatively high concentrations of a hydrophobic component (generally greater than 30% by weight, preferably greater than 40% by weight and most preferably greater than 50% by weight) and/or a composition with a relatively high viscosity, e.g., in excess of 1,000 cps and preferably in excess of 10,000 cps when measured by the Viscosity Test.

For example, in one embodiment, the present invention provides compositions for providing residual antimicrobial efficacy on the skin, in the anterior nares, mucosal tissue, and/or in a wound of a subject, the method includes contacting the skin, mucosal tissue, and/or wound with an antimicrobial composition of the present invention in an amount effective to kill one or more microorganisms.

### DEFINITIONS

The following terms are used herein according to the following definitions.

"Effective amount" means the amount of the one or more components when in a composition, as a whole, provides antimicrobial (including, for example, antiviral, antibacterial, or antifungal) activity when applied in an amount, at a frequency, and for a duration, that reduces, prevents, or eliminates one or more species of microbes such that an acceptable level of the microbe results. Typically, this is a level low enough not to cause clinical symptoms, and is desirably a non-detectable level. It should be understood that in the compositions of the present invention, the concentrations or amounts of the components, when considered separately, may not kill to an acceptable level, or may not kill as broad a spectrum of undesired microorganisms, or may not kill as fast; however, when used together such components provide an enhanced antimicrobial activity (as compared to the same components used alone under the same conditions). Also, it should be understood that (unless otherwise specified) the listed concentrations of the components are for "ready to use" or "as used" compositions. The compositions can be in a concentrated form. That is, certain embodiments of the compositions can be in the form of concentrates that would be diluted by the user with an appropriate vehicle.

"Hydrophilic" or "water-soluble" refers to a material that will disperse or dissolve in deionized water (or other aqueous solution as specified) at a temperature of 23°C in an amount of at least 7% by weight, preferably at least 10% by weight, more preferably at least 20% by weight, even more preferably at least 25% by weight, even more preferably at least 30% by weight, and most preferably at least 40% by weight, based on the total weight of the hydrophilic material and the water. The component is considered dissolved if after thoroughly mixing the compound with water at 60°C for at least 4 hours and allowing this to cool to 23-25°C for 24 hours, and mixing the composition thoroughly it appears uniform clear solution without visible cloudiness, phase separation, or precipitate in a jar having a path length of 4 cm. Typically when placed in 1 x, 1cm cell, the samples exhibit greater than 70% transmission measured in a suitable spectrophotometer at a wavelength of 655 nm. Water dispersible hydrophilic materials disperse in water to form uniform cloudy dispersions after vigorous shaking of a 5% by weight mixture of the hydrophilic component in water. Preferred hydrophilic components are water-soluble.

"Hydrophobic" or "water-insoluble" refers to a material that will not significantly dissolve in deionized water at 23°C. "Not significantly" means that the solubility in water of the material is less than 5% by weight, preferably less than 1% by weight, more preferably less than 0.5% by weight, and even more preferably less than 0.1% by weight, based on the total weight of the hydrophobic material and the water. Solubility can be determined by thoroughly mixing the compound with water at the appropriate concentration at 23°C for at least 24 hours (or at elevated temperature if that is necessary to dissolve the compound), allowing this to sit at 23-25°C for 24 hours, and observing the sample. In a glass jar with a 4cm path length the sample should have evidence of a second phase which can be liquid or solid and may be separated on the top, bottom, or distributed throughout the sample. For crystalline compounds care must be taken to avoid producing a supersaturated solution. The components should be mixed and observed. Cloudiness or presence of a visible precipitate or separate phase indicates that the solubility limit has been exceeded. Typically when placed in 1 x 1cm cell the sample has less than 70% transmission measured in a suitable spectrophotometer at a wavelength of 655 nm. For solubility determinations less than that which can be observed with the naked eye, the solubility is determined using radiolabeled compounds as described under "Conventional Solubility Estimations" in Solubility of Long-Chain Fatty Acids in Phosphate Buffer at pH 7.4, Henrik Vorum, et. al., Biochimica et. Biophysica Acta. 1126 (1992) 135-142.

"Stable" means physically stable or chemically stable, which are both defined in , greater detail below. Preferred compositions are both chemically and physically stable.

"Microorganism" or "microbe" refers to bacteria, yeast, mold, fungi, protozoa, mycoplasma, as well as viruses (including lipid enveloped RNA and DNA viruses).

"Antibiotic" means an organic chemical compound produced by microorganisms that has the ability in dilute concentrations to destroy or inhibit microorganisms and is used to treat infectious disease. This may also encompass semi-synthetic compounds that are chemical derivatives of the compound produced by microorganisms or synthetic compounds that act on very specific biochemical pathways necessary for the cell's survival.

"Antiseptic" means a chemical agent other than the "enhancers," described herein that kills pathogenic and non-pathogenic microorganisms. Preferred antiseptics exhibit at least 4 log reduction of both P. aeruginosa and S. aureus in 60 minutes from an initial inoculum of 1-3 x 10⁷ cfu/ml when tested in Mueller Hinton broth at 35°C at a concentration of 0.25wt% in a Rate of Kill assay using an appropriate neutralizer as described in *The Antimicrobial Activity in vitro of chlorhexidine, a mixture of isothiazolinones (Kathon CG) and cetyl trimethyl ammonium bromide (CTAB),* G. Nicoletti, V. Boghossian, F. Gurevitch, R: Borland and P: Mogenroth, Journal of Hospital Infection, (1993), vol. 23, pp 87-111. Antiseptics generally interfere more broadly with the cellular metabolism and/or the cell envelope. Antiseptics may be small molecule or polymeric. Small molecule antiseptics generally have molecular weights less than about 350 g/mole. Polymeric antiseptics can be much higher in molecular weight.

"Enhancer" means a component that enhances the effectiveness of the antiseptic component such that when the composition less the antiseptic component and the composition less the enhancer component are used separately, they do not provide the same level of antimicrobial activity as the composition as a whole. For example, an enhancer component in the absence of the antiseptic component may not provide any appreciable antimicrobial activity. The enhancing effect can be with respect to the level of kill, the speed of kill, and/or the spectrum of microorganisms killed, and may not be seen for all microorganisms. In fact, an enhanced level of kill is most often seen in Gram negative bacteria such as Escherichia coli. An enhancer may be a synergist such that when combined with the remainder of the composition, the composition as a whole displays an activity that is greater than the sum of the activity of the composition less the enhancer component and the composition less the antiseptic component.

"Mucous membranes," "mucosal membranes," and "mucosal tissue" are used interchangeably and refer to the surfaces of the nasal (including anterior nares, nasoparangyl cavity, etc.), oral (e.g., mouth), outer ear, middle ear, vaginal cavities, and other similar tissues. Examples include mucosal membranes such as buccal, gingival, nasal, ocular, tracheal, bronchial, gastrointestinal, rectal, urethral, ureteral, vaginal, cervical, and uterine mucosal membranes.

"Preservative" as used herein refers to antimicrobials which are incorporated into a composition to prevent biological contamination and/or deterioration of a composition. These are generally present at levels of less than 0.50% by weight and often less than about 0.1% by weight.

"Affliction" means a condition to a body resulting from sickness, disease, injury, bacterial colonization, etc.

"Treat" or "treatment" means to improve the condition of a subject relative to the affliction, typically in terms of clinical symptoms of the condition.

"Decolonization" refers to a reduction in the number of microorganisms (e.g., bacteria and fungi) present in or on tissue that do not necessarily cause immediate clinical symptoms. Examples of decolonization include, but are not limited to, decolonization of the nasal cavity and wounds. Ordinarily fewer microorganisms are present in "colonized tissue" than in "infected tissue." When the tissue is completely decolonized the microorganisms have been "eradicated".

"Subject" and "patient" includes humans, sheep, horses, cattle, pigs, dogs, cats, rats, mice, or other mammal.

"Wound" refers to an injury to a subject which involves a break in the normal skin or mucosal tissue barrier exposing tissue below, which is caused by, for example, lacerations, surgery, burns, damage to underlying tissue such as pressure sores, poor circulation, and the like. Wounds are understood to include both acute and chronic wounds.

The terms "comprises" and variations thereof do not have a limiting meaning where these terms appear in the description and claims.

As used herein, "a," "an," "the," "at least one," and "one or more" are used interchangeably. The term "and/or" means one or all of the listed elements (e.g., preventing and/or treating an affliction means preventing, treating, or both treating and preventing further afflications).

Also herein, the recitations of numerical ranges by endpoints include all numbers subsumed within that range (e.g., 1 to 5 includes 1, 1.5, 2, 2.75, 3, 3.80, 4, 5, etc.).

The above summary of the present invention is not intended to describe each disclosed embodiment or every implementation of the present invention. The description that follows more particularly exemplifies illustrative embodiments: In several places throughout the application, guidance is provided through lists of examples, which examples can be used in various combinations. In each instance, the recited list serves only as a representative group and should not be interpreted as an exclusive list.

The present invention provides antimicrobial (including, e.g., antiviral, antibacterial, and antifungal) compositions. These compositions include one or more antiseptics selected from the group consisting of C6-C14 alkyl carboxylic acids and alkyl carboxylate ester carboxylic acids, and antimicrobial natural oils in sufficient concentration (typically 0.20%, preferably greater than 0.30%, and more preferably greater than 0.50% by weight) which when applied to mammalian tissue for an adequate time, for an adequate frequency, and in an adequate dose is capable of decolonizing or eradicating microorganisms from the tissue. The compositions also include one or more hydrophilic compounds, and one or more hydrophobic compounds and may include one or more surfactants.

Such compositions preferably adhere well to bodily tissues (e.g., skin, mucosal tissue, and wounds) and thus are very effective topically. Importantly, the compositions, however, are not bioadhesive and thus will not bond tissue together. Thus, the present invention provides a wide variety of uses of the compositions. Particularly preferred methods involve topical application, particularly to mucosal tissues (i.e., mucous membranes including the anterior nares and other tissues of the upper respiratory tract), as well as skin (e.g., skin lesions) and wounds.

For certain applications in which broad spectrum antimicrobial activity is desired, compositions containing multiple antiseptics can be used. In other applications in which limited antimicrobial activity is desired, compositions containing an antiseptic with limited spectrum may be employed. For example, in certain situations it may be desirable to kill or inactivate only one type or a few types of microorganism as opposed to all the microorganisms present. For example, as shown in the Examples, compositions comprising lauric acid in a petrolatum vehicle have activity against Methicillin Resistant *Staphylococcus Aureus (MRSA)* (Gram positive microorganisms), but only limited activity against E. coli (Gram negative microorganisms), and thus may be more useful in situations where it is desirable to kill mainly Gram positive organisms such as in nasal decolonization, treatment of impetigo and in other topical infections caused primarily by Gram positive organisms.

Compositions of the present invention can be used to provide effective topical antimicrobial activity and thereby treat and/or prevent a wide variety of afflications. For example, they can be used in the treatment and/or prevention of afflictions that are caused, or aggravated by, microorganisms (e.g., Gram positive bacteria, Gram negative bacteria, fungi, protozoa, mycoplasma, yeast, viruses, and even lipid-enveloped viruses) on mammalian tissue, i.e., skin and/or mucous membranes, such as those in the nose (anterial nares, nasopharangyl cavity, nasal cavities, etc.), outer ear, middle ear, mouth, rectum, vagina, or other similar tissue. Particularly relevant organisms that cause or aggravate such afflications include *Staphylococcus spp., Streptococcus spp., Pseudomonas spp., Enterococcus spp.,* and *Esherichia spp.,* bacteria, as well as herpes virus, *Aspergillus spp., Fusarium spp., Candida spp* and combinations thereof. Particularly virulent organisms include *Staphylococcus aureus* (including resistant strains such as Methicillin Resistant *Staphylococcus Aureus (MRSA), Staphylococcus epidermidis, Streptococcus pneumoniae, Enterococcus faecalis, Vancomycin Resistant Enterococcus (VRE), Pseudomonas auerginosa, Escherichia coli, Aspergillus niger, Aspergillus fumigatus, Aspergillus clavatus, Fusarium solani, Fusarium oxysporum, Fusarium chlamydosporum, Candida albicans, Candida glabrata,* and *Candida krusei.*

Compositions of the present invention can be used for the prevention and/or treatment of one or more microorganism-caused infections or other afflictions. In particular, compositions of the present invention can be used for preventing and/or treating one or more of the following: skin lesions, conditions of the skin such as impetigo, eczema, psorasis, diaper rash in infants as well as incontinent adults, inflammation around ostomy devices, shingles, and bacterial infections in open wounds (e.g., cuts, scrapes, burns, lacerations, chronic wounds); necrotizing faciitis; infections of the outer ear; acute or chronic otitis media (middle ear infection) caused by bacterial, viral, or fungal contamination; fungal and bacterial infections of the vagina or rectum; vaginal yeast infections; bacterial rhinitis; ocular infections; cold sores; genital herpes; colonization by *Staphylococcus aureus* in the anterior nares (e.g., prior to surgery or hemodialysis); mucositis (i.e., inflammation as opposed to infection of a mucous membrane typically induced by non-invasive fungus); chronic sinusitis (e.g., that caused by bacterial or viral contamination); non-invasive fungus-induced rhinosinusitis; chronic colitis; Crohn's disease; bums; napkin rash; tinea pedis (i.e., athlete's foot); tinea curis (i.e., jock itch); tinea corporis (i.e., ringworm); candidiasis; strep throat, strep pharyngitis, and other Group A *Streptococci* infections; rosacea (often called adult acne); common cold; and respiratory afflictions (e.g., asthma). In sum, compositions of the present invention can be used for preventing and/or treating a wide variety of topical afflictions caused by microbial infection (e.g., yeast, viral, bacterial infections).

Compositions of the present invention can be used on a wide variety of surfaces. For example, they can be used on mammalian tissue (e.g., skin, mucosal tissue, chronic wounds, acute wounds, bums). They can also be delivered from swabs, cloth, sponges, foams and non-woven and paper products (e.g., paper towels and wipes), for example
where they are used to deliver a significant portion of the antiseptic composition to the tissue. By "significant portion" it is meant that enough composition is applied and allowed to remain on the tissue when applied in a dose, at a frequency, and in an amount sufficient to reduce or eliminate the microorganisms on or in the tissue.

Thus, the present invention also provides various methods of use of compositions of the present invention. Various embodiments of the present invention include: a method of preventing an affliction caused, or aggravated by, a microorganism on skin and/or a mucous membrane; a method of decolonizing at least a portion of the nasal cavities, anterior nares, and/or nasopharynx of a subject of microorganisms; a method of eradicating microorganisms from at least a portion of the nasal cavities, anterior nares, and/or nasopharynx of a subject; a method of treating a middle ear infection in a subject (by introduction into the middle ear through the Eustachian tube, and/or the tympanic membrane by diffusion or direct injection); a method of treating chronic sinusitis in a subject (by treating at least a portion of the respiratory system, particularly the upper respiratory system, including the nasal cavities, anterior nares, and/or nasopharynx); a method of treating impetigo on the skin of a subject; a method of treating and/or preventing an infection on the skin, mucosal tissue, and/or wound of a subject; a method of treating a burn; a method of killing or inactivating microorganisms (e.g., killing bacteria and/or fungi, or inactivating viruses); a method for-providing residual antimicrobial efficacy (e.g., antibacterial, antfungal, and/or antiviral efficacy) that results from leaving a residue or imparting a condition on a surface (such as skin, mucosal tissue, wound, and/or medical device that contacts such surfaces) that remains effective and provides significant antimicrobial activity. Not all of the antiseptics disclosed herein are useful for all of these conditions. Suitable indications for each antiseptic are discussed below.

It should be understood that compositions of the present invention can be used in situations in which there are no clinical indications of an affliction. For example, compositions of the present invention can be used in methods of decolonizing at least a portion of the nasal cavities (i.e., space behind the vestibule of the nose), anterior nares (i.e., the opening in the nose to the nasal cavities, also referred to as the external nares), and/or nasopharynx (i.e., the portion of the pharynx, i.e., throat, that lies above the point of food entry into the pharynx) of a subject of microorganisms. A suitable in-vivo model to test for the effectiveness of compositions to decolonize the anterior nares has been established and is described by K. Kiser et al., Infect and Immunity, 67(10), 5001-5006 (1999). Compositions of the present invention can also be used to decolonize microorganisms from wounds. Also disclosed in the example section is an in-vitro model that places microorganisms in contact with a static coating of the antimicrobial composition. This test method is suitable for comparing the potential efficacy of compositions of the present invention for most topical antiseptic applications, including nasal decolonization.

Decolonization methods using compositions of the present invention are particularly useful in immunocompromised patients (including oncology patients, diabetics, HIV patients, transplant patients an the like), particularly for fungi such as *Aspergillus spp.* and *Fusarium spp.*

In particular, compositions of the present invention can be used in chronic wounds to eliminate methicillin-resistant *Staphylococcus aureus* and vancomycin resistant *enterococcus,* which may or may not show clinical signs of infection such as inflammation, pus, exudate, etc. Also, it is of significance to note that certain compositions of the present invention can kill lipid-enveloped viruses, which can be very difficult to kill and can cause shingles (Herpes), chronic sinusitis, otitis media, and other local diseases.

Those of ordinary skill in the art will readily determine when a composition of the present invention provides antimicrobial activity using assay and bacterial screening methods well known in the art. One readily performed assay involves exposing selected known or readily available viable microorganism strains, such as *Enterococcus spp., Aspergillus spp., Escherichia spp., Staphylococcus spp., Streptococcus spp., Pseudomonas spp.,* or *Salmonella spp.,* to a test composition at a predetermined bacterial burden level in a culture media at an appropriate temperature.' For the preferred compositions of the present invention, testing is most conveniently done by the Antimicrobial Efficacy Test described in the Examples Section. Briefly, the antimicrobial composition is coated onto a sterile surface and a bacterial suspension is distributed directly on the surface of the composition. After a sufficient contact time, the sample containing the exposed bacteria is collected, placed in neutralizing broth, a sample is taken and diluted, and plated out on agar. The plated sample is incubated at an appropriate temperature and humidity for forty-eight hours and the number of viable bacterial colonies growing on the plate is counted. Once colonies have been counted the reduction in the number of bacteria caused by the test composition is readily determined. Bacterial reduction is generally reported as log₁₀ reduction determined by the difference between the log₁₀ of the initial inoculum count and the log₁₀ of the inoculum count after exposure. Preferred compositions of the present invention have an average of at least a 2 log reduction in test bacteria in 10 minutes, and preferably in 2.5 minutes.

Many of the preferred compositions were tested as described in the Examples Section for antimicrobial activity against MRSA (Gram positive, ATCC Number 16266) and *E. coli* (Gram negative, ATCC Number 11229). Preferred compositions of the present invention also exhibit very rapid antimicrobial activity. As shown in the Examples Section, preferred formulations are able to achieve an average log reduction of at least 4 log against at leat one of these two organisms after a 10 minute exposure and preferably after a 2.5 minute exposure. More preferred compositions are able to achieve an average log reduction of at least 5 log and even more preferred at least 6 log against at least one of these three organisms after a 10 minute exposure and preferably after a 2.5 minute exposure.

For residual antimicrobial efficacy, compositions of the present invention preferably maintain an average log reduction of at least 1 log, more preferably at least 1.5 log, and even more preferably at least 2 log, for at least 1 hour, more preferably at least 3 hours, and even more preferably at least 24 hours after application to an affected site or after testing the composition on the forearm of a subject. To test this, a composition was applied to the forearm of a subject as a uniform wet coating in an amount of approximately 4 milligrams per square centimeter (mg/cm²) to the forearm of a healthy subject and allowed to thoroughly dry, or set in the case of compositions with no volatile components (typically a minimum of 10 minutes) over an area of approximately 5 x 5 cm. The composition was gently washed with 23°C normal saline (0.9% by weight sodium chloride). The saline washed site was exposed to a known quantity of bacteria in an innoculum of about 10⁶ bacteria/ml (typically *Staphylococcus epidermidis* or *E. coli)* for 30 minutes. The bacteria were recovered and treated with an effective neutralizer and incubated to quantify the bacteria remaining. Particularly preferred compositions retain at least 1 log reduction and preferably at least 2 log reduction of bacteria after a gentle rinse with 500 ml saline poured over the site by placing the saline container as close the the site as possible so as to not have the saline fall onto the site.

Importantly, certain embodiments of the present invention have a very low potential for generating microbial resistance. For example, preferred compositions of the present invention have an increase in the ratio of final to initial MIC levels (i.e., minimum inhibitory concentration) of less than 16, more preferably less than 8, and even more preferably less than 4. Such an emergence of resistance assay should be carried out such that the microorganisms are subjected initially to sub MIC levels (e.g., ½ the MIC) of antiseptic and after 24 hours the microorganisms passed into broth containing twice the concentration of antiseptic. This is repeated for 8 days and each day microorganisms are removed to determine the new MIC. Thus, such low resistance forming compositions can be applied multiple times over one or more days to treat topical infections or to eradicate unwanted bacteria (such as nasal colonization of *Staphylococcus aureus*).

Preferred compositions of the present invention contain an effective amount of antimicrobial to rapidly kill or inactivate microorganisms on skin, skin lesions, and mucosal membranes. In certain embodiments, essentially all the microorganisms are eradicated or inactivated using one or more doses within five days, preferably within three days, more preferably two days, and most preferably within 24 hours.

Preferred compositions of the present invention have a generally low irritation level for skin, skin lesions, and mucosal membranes (including the anterior nares, nasal cavities, nasopharangyl cavity and other portions of the upper respiratory tract). For example, certain preferred compositions of the present invention are no more irritating than BACTROBAN ointment (on skin) or BACTROBAN NASAL (in the anterior nares) products available from Glaxo Smith Kline.

Preferred compositions of the present invention are substantive for relatively long periods of time to ensure adequate efficacy. For example, certain compositions of the present invention remain at the site of application with antimicrobial activity for at least 1 hour, preferably at least 4 hours, and more preferably at least 8 hours. Substantivity can be determined by swabbing the site after a predetermined time and testing for the antimicrobial active by a suitable analytical technique such as gas chromatography (GC) or high performance liquid chromatography (HPLC).

Preferred compositions of the present invention are physically stable. As defined herein "physically stable" compositions are those that do not significantly change due to substantial precipitation, crystallization, phase separation, and the like, from their original condition during storage at 23°C for at least 3 months, and preferably for at least 6 months. Particularly preferred compositions are completely physically stable if a 10-milliliter (10-ml) sample of the composition when placed in a 15-ml conical-shaped graduated plastic centrifuge tube (Coming) and centrifuged at 2275 x g (e.g., at 3,000 revolutions per minute (rpm) for 10 minutes using a Labofuge B, model 2650 manufactured by Heraeus Sepatech GmbH, Osterode, West Germany) or similar centrifuge at a centrifugal force of 2275 x g, has no visible phase separation in the bottom or top of the tube. Phase separation of less than 0.5ml is also considered stable as long as there is no other sign of physical separation in the sample.

Preferred compositions of the present invention exhibit good chemical stability. This can be especially a concern with compounds that may hydrolyze or undergo heat and/or light degradation. The most preferred compositions retain an average of at least 97% of the antimicrobial component after aging for 4 weeks at 40°C in a sealed container beyond the initial 5-day equilibration period at 23°C. The percent retention is understood to mean the weight percent of antimicrobial component retained. This is determined by comparing the amount remaining in a sample aged (i.e., aged beyond the initial 5-day equilibration period) in a sealed container that does not cause degradation, to the actual measured level in an identically prepared sample (preferably from the same batch) and allowed to sit at 23°C for five days. The level of antimicrobial component is preferably determined using gas chromatography or high performance liquid chromatography.

Generally, the compositions of this invention may be in one of the following forms:
- A hydrophobic ointment: The compositions are formulated with a hydrophobic base (e.g., petrolatum, thickened or gelled water insoluble oils and the like) and optionally having a minor amount of a water soluble phase.
- An oil in water emulsion: The compositions may be formulations in which the antiseptic is emulsified into an emulsion comprising a discrete phase of a hydrophobic component and a continuous aqueous phase comprising water and optionally one or more polar hydrophilic carrier as well as salts, surfactants, emulsifiers, or other components. These emulsions may comprise water soluble or water swellable polymers as well as one or more emulsifiers that help to stabilize the emulsion. These emulsions generally have higher conductivity values as described in U.S. Serial No. 09/966,511.
- A water in oil emulsion: The compositions may be formulations in which the antiseptic is incorporated into an emulsion comprising a continuous phase of a hydrophobic component and an aqueous phase comprising water and optionally one or more polar hydrophilic carrier as well as salts or other components. These emulsions may comprise oil soluble or oil swellable polymers as well as one or more emulsifiers that help to stabilize the emulsion.
- Thickened Aqueous gels: These systems are comprised of an aqueous phase which has been thickened to achieve a viscosity in excess of 500 cps and preferably greater than 5000 cps. Most preferred systems have a viscosity in excess of 10,000 cps, more preferably greater than 25,000 cps and most preferably greater than 50,000 cps. The viscosity is determined using the Viscosity Test described herein. These systems comprise the antiseptics described here in and are thickened by suitable natural, modified natural, or synthetic polymers as described below. The thickened aqueous gels can also be thickened using suitable emulsifiers such as alkyl alcohols and polyethoxylated alkyl chain surfactants that effectively thicken the composition. Examples include the Polawax, Behenyl TMS, Crodaphos CES, Cosmowax, and Crothix systems from Croda Inc.
- Hydrophilic gels: These are systems in which the continuous phase is comprised of at least one water soluble hydrophilic component other than water. The formulations may optionally also contain water up to about 20% by weight. Higher concentrations may be suitable in some compositions. Suitable hydrophilic components include one or more glycols (such as glycerin, propylene glycol, butylenes glycol), polyethylene glycols (PEG), random or block copolymers of ethylene oxide, propylene oxide, and/or butylenes oxide, polyalkoxylated surfactants having one or more hydrophobic moieties per molecule, silicone copolyols, and combinations thereof. One skilled in the art will recognize that the level of ethoxylation must be sufficient to render the hydrophilic component water soluble or water dispersible at 23C. In most embodiments, the water content is less than 10% and more preferably less than about 5% by weight of the composition.

In most embodiments, the compositions have a viscosity of at least 20 cps, preferably greater than 100 cps, more preferably greater than 1000 cps, even more preferably greater than 10,000 cps and most preferably greater than 25,000 cps when measured by the Viscosity Test described herein. Higher viscosities are preferred to reduce migration as well as to provide substantivity (resistance to removal by fluids) to ensure longterm antimicrobial activity. Most preferred compositions have viscosities in excess of 50,000 cps and most preferably in excess of 100,000 cps at 23-25°C when measured by the Viscosity Test. Most preferred compositions meet these viscosity values even after heating to 32°C, 35°C or as high as 37°C to ensure when in contact with mammalian tissue the compositions remain substantive.

### Antiseptic Component

The antiseptic component is that component of the composition that provides at least part of the antimicrobial activity. That is, the antiseptic component has at least some antimicrobial activity for at least one microorganism. It is generally considered the main active component of the compositions of the present invention. The antiseptic component includes an effective amount of one or more antiseptics selected from the group consisting of C6-C14 alkyl carboxylic acids and alkyl carboxylate ester carboxylic acids, antimicrobial natural oils, and compatible combinations thereof.
C6-C14 Alkyl Carboxylic acids and Alkyl Carboxylate Ester Carboxylic acids: This class of antimicrobial antiseptics includes C6-C14 and preferably C8-C12 straight chain or branched alkyl carboxylic acids such as heptanoic, capric, caprylic, undecylenic, and lauric acids. These are often referred to as fatty acids. As used herein the term "fatty" includes both even and odd number of carbon atoms in alkyl acids which may be linear or branched. Also included are C8-C22 mono- or polyunsaturated fatty acids. Examples include oleic, linoleic, linolenic, and arachidonic acids. Also included within this class are esters of these carboxylic acids with hydroxyfunctional alkyl acids (alkyl carboxylate esters of carboxylic acids) such as lauroyl lactylate, capryloil lactylate or caproyl lactylate. The alkyl carboxylate ester carboxylic acids comprise a C6-C14, preferably C8-C12 alkyl group. Most conveniently, these are formed by esterification of a C6-C 14 saturated linear or branched alkylcarboxylic acid or a C8-C22 mono- or polyunsaturated fatty acid with a hydroxyfunctional alkyl carboxylic acid. A commercially available example of an alkyl carboxylate ester of an alkyl carboxylic acid is Pationic 122A (caproyl lactylate) available from RITA Corp. Another preferred compound of this class is lauroyl lactylate. It is preferred to formulate these antiseptics in the presence of a hydrophobic component and/or an emulsifier/surfactant. These compounds are typically added to the formulations in amounts of 0.5% by weight, preferably 1% by weight, and most preferably 2% by weight. In most embodiments, the compounds are added in amounts of no greater than 8wt%, more preferably no greater than 6wt%, and most preferably no greater than 4wt%.

At least a portion of the carboxylic acid preferably is present in the acid or protonated form. This form has significantly greater activity than the neutralized salt form. Since these acids can also be relatively irritating they are preferably formulated in compositions based on hydrophobic vehicles such as emollient oils or petrolatum which may optionally contain a hydrophilic component. The pH of aqueous compositions (or the aqueous phase of the compositions), formulated with these antiseptics typically range from 3-8 and most preferably from 3 to 6.
Natural oil derived antiseptics; This class includes oils and oil extracts from plants such as Tea Tree oil, grape fruit seed extract, Aspidium extract (phloro, lucinol containing extract); barberry extract (berberine chloride); bay sweet extract; bayberry bark extract (myricitrin); cade oil; CAE (available from Ajinomoto, located in Teaneck, N.J.); cajeput oil; caraway oil; cascarilla bark (sold under the tradename ESSENTIAL OIL); cedarleaf oil; chamomille; cinnamon oil; citronella oil; clove oil; German chamomile oil; giant knotweed; lemon balm oil; lemon grass; olive leaf extract (available from Bio Botanica); parsley; patchouly oil; peony root; pine needle oil; PLANSERVATIVE (available from Campo Research); rose geranium oil; rosemary; sage, and the like as well as mixtures thereof. Particularly preferred are tea tree oil (cajeput oil) and grapefruit seed extract.

These compounds may be relatively water insoluble and thus it may be preferred to formulate these compounds in the presence of a hydrophobic component and/or an emulsifier/surfactant, in an emulsion (water in oil or oil in water), or in a hydrophilic vehicle. These compounds are typically added to the formulations at 0.5-8%, preferably 1-6%, and most preferably 2-4% by weight. Significantly higher levels may be required in hydrophobic components that are good solvents for the antiseptics to ensure some of the antiseptic is available to kill the microorganisms. Preferred compositions are formulated free of polyethylene glycol with a MW greater than about 1500 daltons, and more preferably greater than 600 daltons, which may reduce the activity. In most embodiments, the compositions are those based on hydrophobic vehicles (such as petrolatum) with an optional hydrophilic component and/or water in oil emulsions. The pH of compositions formulated with these antiseptics typically range from 3-9 and most preferably from 3.5-7.

The compositions of the present invention include one or more antiseptics at a suitable level to produce the desired result. Such compositions preferably include a total amount of antiseptic of at least 0.2 percent by weight (wt-%), more preferably at least 0.25 wt-%, even more preferably at least 0.35 wt-%, even more preferably at least 0.5 wt-%, and even more preferably at least 1, 2, or even 3 wt-%, based on the total weight of the "ready to use" or "as used" composition. In a preferred embodiment, the antiseptic(s) are present in a total amount of no greater than 20 wt-%, more preferably no greater than 15 wt-%, even more preferably no greater than 10 wt-%, and even more preferably no greater than 5 wt-%, based on the "ready to use" or "as used" composition. Certain compositions may be higher in concentration if they are intended to be diluted prior to use.

The antiseptics of this invention may be used alone, in combination, or with other antiseptics in order to effectively kill microorganisms on tissue. Additional antiseptics for use with those described herein include halogenated phenols, diphenyl ethers, bisphenols (including but not limited to p-chloro m-xylenol (PCMX) and triclosan), phenols, resorcinols and its derivatives, anilides, and combinations thereof, provided in Applicants' copending application entitled "Phenolic Antiseptic Compositions and Methods of Use," U.S. Serial No. 10/936,171, filed September 7, 2004; chlorhexidine and its salts such as digluconate, diacetate, dimethosulfate, and dilactate salts; polymeric quaternary ammonium compounds such as polyhexamethylenebiguanide; silver and various silver complexes; small molecule quaternary ammonium compounds such as benzalkoium chloride and alkyl substituted derivatives; di-long chain alkyl (C6-C 18) quaternary ammonium compounds; cetylpyridinium halides and their derivatives; benzethonium chloride and its alkyl substituted derivatives; and octenidine provided in Applicants' copending application entitled "Cationic Antiseptic Compositions and Methods of Use," U.S. Serial No. 10/936,135, filed September 7, 2004.

Certain combinations of antiseptics may be particularly useful while others may result in unstable formulations or inactivation of the antimicrobial activity. For example, combination of cationic antiseptics such as biguanides and bisbiguanides, polymeric quaternary ammonium compounds, quaternary ammonium compounds, and silver may be incompatible with alkyl carboxylic acids. On the other hand, other antiseptic combinations may produce a synergistic or enhancing effect. For example, C6 and higher fatty acids may enhance the activity of peroxides as well as the fatty acid monoglyceride antiseptics described below.

In certain embodiments, the antiseptics of this invention may optionally be combined with an effective amount of an antimicrobial lipid antiseptic comprising a (C7-C14)saturated fatty acid ester of a polyhydric alcohol, a (C8-C22)unsaturated fatty acid ester of a polyhydric alcohol, a (C7-C14)saturated fatty ether of a polyhydric alcohol, a (C8-C22)unsaturated fatty ether of a polyhydric alcohol, an alkoxylated derivative thereof, or combinations thereof. The alkoxylated derivatives typically have less than 5 moles of alkoxide per mole of polyhydric alcohol. Generally, for polyhydric alcohols other than sucrose, the esters comprise monoesters and the ethers comprise monoethers, and for sucrose the esters comprise monoesters, diesters, or combinations thereof, and the ethers comprise monoethers, diethers, or combinations thereof. Useful antiseptics of this class are further described in "Antimicrobial Compositions and Methods of Use," U.S. Serial No. 10/659,571. As used herein the term "fatty" refers to alkyl and alkylene hydrocarbon chains of odd or even number of carbon atoms from C6-C18.

Alternatively, the antimicrobial lipid can be a (C8-C12)fatty alcohol ester of a (C2-C8)hydroxycarboxylic acid (also often referred to as a (C2-C8)hydroxycarboxylic acid ester of a (C8-C12)fatty alcohol), a (C8-C22)mono- or poly-unsaturated fatty alcohol ester of a (C2-C8)hydroxycarboxylic acid (also often referred to as a (C2-C8)hydroxycarboxylic acid ester of a (C8-C22)mono- or poly-unsaturated fatty alcohol), or alkoxylated derivatives thereof.. The alkoxylated derivatives have less than 5 moles of alkoxide per mole of polyhydric alcohol or hydroxyl acid. The hydroxycarboxylic acid moiety can include aliphatic and/or aromatic groups. For example, fatty alcohol esters of salicylic acid are possible. Useful antiseptics of this class are further described in applicants' copending application "Antimicrobial Compositions and Methods" U.S. Serial No. 60/660,594, filed on March 10, 2005.

As used herein, a "fatty alcohol" is an alkyl or alkylene monofunctional alcohol having an even or odd number of carbon atoms and a "fatty acid" is a alkyl or alkylene monofunctional carboxylic acid having an even or odd number of carbon atoms.

To achieve rapid antimicrobial activity, formulations may incorporate one or more antiseptics in the composition approaching or preferably exceeding the solubility limit in the hydrophobic phase. While not intended to be bound by theory, it appears that antiseptics that preferably partition into the hydrophobic component are not readily available to kill microorganisms that are in or associated with an aqueous medium. In most compositions, the antiseptic is preferably incorporated in at least 60%, preferably at least 75%, more preferably at least 100% and most preferably at least 120% of the solubility limit of the hydrophobic component at 23°C. This in conveniently determined by making the formulation without the antiseptic, separating the phases (e.g., by centrifugation or other suitable separation technique) and determining the solubility limit by addition of progressively greater levels of the antiseptic until precipitation occurs. One skilled in the art will realize that creation of supersaturated solutions must be avoided for an accurate determination. For example, we have found that compositions using hydrophobic vehicles that contain Tea Tree Oil are significantly more active above the solubility limit.

### Enhancer Component

Compositions of the present invention include an enhancer to enhance the antimicrobial activity. The activity enhancement may be especially useful against Gram negative bacteria, such as *E. coli* and *Psuedomonas sp.* The enhancer chosen preferably effects the cell envelope of the bacteria. While not bound by theory, it is presently believed that the enhancer functions by allowing the antiseptic to more easily enter the cell cytoplasm and/or by facilitating disruption of the cell envelope. The enhancer component includes an alpha-hydroxy acid, a beta-hydroxy acid, a chelating agent a (C1-C4)alkyl carboxylic acid, a (C6-C12)aryl carboxylic acid, a (C6-C12)aralkyl carboxylic acid, a (C6-C16)alkaryl carboxylic acid, a phenolic compound (such as certain antioxidants and parabens), a (C1-C10) alkyl alcohol, or a glycol ether (i.e., ether glycol). Various combinations of enhancers can be used if desired.

In some embodiments, other enhancers may be useful, such as the siderophores and iron-bonding proteins described in U.S. Serial No. 10/936,949, filed September 8, 2004 entitled "Antimicrobial Compositions and Methods"; and the sugar and/or alcohols as described in U.S. Serial No. 60/660,830, filed March 10, 2005 entitled "Methods of Reducing Microbial Contamination."

The alpha-hydroxy acid, beta-hydroxy acid, and other carboxylic acid enhancers are preferably present in their protonated, free acid form. It is not necessary for all of the acidic enhancers to be present in the free acid form, however, the preferred concentrations listed below refer to the amount present in the free acid form. Additonal, non-alpha hydroxy acid, beta-hydroxy acid or other carboxylic acid enhancers, may be added in order to acidify the formulation or buffer it at a pH to maintain antimicrobial activity. Furthermore, the chelator enhancers that include carboxylic acid groups are preferably present with at least one, and more preferably at least two, carboxylic acid groups in their free acid form. The concentrations given below assume this to be the case. While the non-ionic enhancers may be useful for all of the antiseptic classes of this invention, the anionic enhancers such as the carboxylic acids and chelators may not be compatible with other components, such as cationic antiseptics. Chelator enhancers may also comprise phosphate or phosphonic acid groups. If precipitation occurs due to interaction with other composition components, alternative enhancers should be considered.

One or more enhancers may be used in the compositions of the present invention at a suitable level to produce the desired result. In a preferred embodiment, they are present in a total amount greather than 0.01 wt-%, preferably in an amount greater than 0.1 wt%, more preferably in an amount greater than 0.2 wt%, even more preferably in an amount greater than 0.25 wt% and most preferably in an amount greater than about 0.4 wt% based on the total weight of the ready to use composition. In a preferred embodiment, they are present in a total amount of no greater than 20 wt-%, based on the total weight of the ready to use composition. Such concentrations typically apply to alpha-hydroxy acids, beta-hydroxy acids, other carboxylic acids, chelating agents, phenolics, ether glycols, (C5-C10)monohydroxy alcohols. Generally, higher concentrations are needed for (C1-C4)monohydroxy alcohols, as described in greater detail below.

The alpha-hydroxy acid, beta-hydroxy acid, and other carboxylic acid enhancers, as well as chelators that include carboxylic acid groups, are preferably present in a concentration of no greater than 100 milliMoles per 100 grams of formulated composition. In most embodiments, alpha-hydroxy acid, beta-hydroxy acid, and other carboxylic acid enhancers, as well as chelators that include carboxylic acid groups, are preferably present in a concentration of no greater than 75 milliMoles per 100 grams, more preferably no greater than 50 milliMoles per 100 grams, and most preferably no greater than 25 milliMoles per 100 grams of formulated composition.

The total concentration of the enhancer component relative to the total concentration of the antiseptic component is preferably within a range of 10:1 to 1:300, and more preferably 5:1 to 1:10, on a weight basis.

An additional consideration when using an enhancer is the solubility and physical stability in the compositions. Many of the enhancers discussed herein are insoluble in preferred hydrophobic components such as mineral oil or petrolatum. It has been found that the addition of a minor amount (typically less than 30 wt-%, preferably less than 20 wt-%, and more preferably less than 12 wt-%) of a hydrophilic component not only helps dissolve and physically stabilize the composition but improves the antimicrobial activity as well. These hydrophilic components are described below. Alternatively, the enhancer may be present in excess of the solubility limit provided that the composition is physically stable. This may be achieved by utilizing a sufficiently viscous composition that stratification (e.g., settling or creaming) of the antiseptic does not appreciably occur.

Alpha-hydroxy Acids. An alpha-hydroxy acid is typically a compound represented by the formula:

R⁵(CR⁶OH)ₙCOOH

wherein: R⁵ and R⁶ are each independently H, a (C1-C8)alkyl group (straight, branched, or cyclic), a (C6-C12)aryl group, a (C6-C12)aralkyl group or (C6-C12)alkaryl group (wherein the alkyl group of the aralkyl or alkaryl is straight, branched, or cyclic), wherein R⁵ and R⁶ may be optionally substituted with one or more carboxylic acid groups; and n = 1-3, preferably, n = 1-2.

Exemplary alpha-hydroxy acids include, but are not limited to, lactic acid, malic acid, citric acid, 2-hydroxybutanoic acid, mandelic acid, gluconic acid, glycolic acid (i.e., alpha-hydroxyethanoic acid), tartaric acid, ascorbic acid, alpha-hydroxyoctanoic acid, and alpha hydroxycaprylic acid, as well as derivatives thereof (e.g., compounds substituted with hydroxyls, phenyl groups, hydroxyphenyl groups, alkyl groups, halogens, as well as combinations thereof). Preferred alpha-hydroxy acids include lactic acid, malic acid, and mandelic acid. These acids may be in D, L, or DL form and may be present as free acid, lactone, or partial salts thereof. All such forms are encompassed by the term "acid." Preferably, the acids are present in the free acid form. In certain preferred embodiments, the alpha-hydroxy acids useful in the compositions of the present invention are selected from the group consisting of lactic acid, mandelic acid, and malic acid, and mixtures thereof. Other suitable alpha-hydroxy acids are described in U.S. Pat. No. 5,665,776 (Yu).

One or more alpha-hydroxy acids may be used in the compositions of the present invention at a suitable level to produce the desired result. In a preferred embodiment, they are present in a total amount of at least 0.25 wt-%, more preferably, at least 0.5 wt-%, and even more preferably, at least 1 wt-%, based on the total weight of the ready to use composition. In a preferred embodiment, they are present in a total amount of no greater than 10 wt-%, more preferably, no greater than 5 wt-%, and even more preferably, no greater than 3 wt-%, based on the total weight of the ready to use composition. Higher concentrations may become irritating.

The ratio of alpha-hydroxy acid enhancer to total antimicrobial antiseptic is preferably at most 10:1, more preferably at most 5:1, and even more preferably at most 1:1. The ratio of alpha-hydroxy acid enhancer to total antimicrobial antiseptic is preferably at least 1:20, more preferably at least 1:12, and even more preferably at least 1:5. Preferably the ratio of alpha-hydroxy acid enhancer to total antimicrobial antiseptic is within a range of 1:12 to 1:1.

Beta-hydroxy Acids. A beta-hydroxy acid is typically a compound represented by the formula:

R⁷(CR⁸OH)ₙ(CHR⁹)ₘCOOH

or 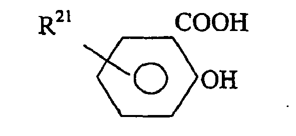 wherein: R⁷, R⁸, and R⁹ are each independently H, a (C1-C8)alkyl group (saturated straight, branched, or cyclic group), a (C6-C12)aryl group, a (C6-C12)aralkyl group or (C6-C12)alkaryl group (wherein the alkyl group of the aralkyl or alkaryl is straight, branched, or cyclic), wherein R⁷ and R⁸ may be optionally substituted with one or more carboxylic acid groups; m = 0 or 1; n = 1-3 (preferably, n = 1-2); and R²¹ is H, (C1-C4)alkyl or a halogen.

Exemplary beta-hydroxy acids include, but are not limited to, beta-hydroxybutanoic acid, 3-hydroxybutanoic acid, tropic.acid, and trethocanic acid. In certain preferred embodiments, the beta-hydroxy acids useful in the compositions of the present invention are selected from the group consisting of salicylic acid, beta-hydroxybutanoic acid, and mixtures thereof. Other suitable beta-hydroxy acids are described in U.S. Pat. No. 5,665,776 (Yu).

One or more beta-hydroxy acids may be used in the compositions of the present invention at a suitable level to produce the desired result. In a preferred embodiment, they are present in a total amount of at least 0.1 wt-%, more preferably at least 0.25 wt-%, and even more preferably at least 0.5 wt-%, based on the total weight of the ready to use composition. In a preferred embodiment, they are present in a total amount of no greater than 10 wt-%, more preferably no greater than 5 wt-%, and even more preferably no greater than 3 wt-%, based on the total weight of the ready to use composition. Higher concentrations may become irritating.

The ratio of beta-hydroxy acid enhancer to total antiseptic component is preferably at most 10:1, more preferably at most 5:1, and even more preferably at most 1:1. The ratio of beta-hydroxy acid enhancer to total antiseptic component is preferably at least 1:20, more preferably at least 1:15, and even more preferably at least 1:10. Preferably the ratio of beta-hydroxy acid enhancer to total antiseptic component is within a range of 1:15 to 1:1.

In systems with low concentrations of water, or that are essentially free of water, esterification may be the principle route of loss of the enhancer by reaction with, for example, the antiseptic or a hydroxyl functional hydrophilic component. Thus, certain alpha-hydroxy acids (AHA) and beta-hydroxy acids (BHA) are particularly preferred since these are believed to be less likely to esterify by reaction of the hydroxyl group of the AHA or BHA. For example, salicylic acid may be particularly preferred in certain formulations since the phenolic hydroxyl group is much more acidic than an aliphatic hydroxyl group and thus much less likely to react. Other particularly preferred compounds in anhydrous or low-water content formulations include lactic, mandelic, malic, citric, tartaric, and glycolic acid. Benzoic acid and substituted benzoic acids which do not comprise a hydroxyl group, while not an hydroxyl acid, are also preferred due to a reduced tendency to form ester groups.

Other Carboxylic Acids. Carboxylic acids other than alpha- and beta-carboxylic acids namely a (C1-C4)alkyl carboxylic acid, a (C6-C12)aralkyl carboxylic acid, or a (C6- C16)alkaryl carboxylic acid are suitable for use in the enhancer component. Exemplary acids include, but are not limited to, acetic acid, propionic acid, benzoic acid, benzylic acid, nonylbenzoic acid, and the like. Particularly preferred is benzoic acid.

One or more carboxylic acids other than alpha- or beta-hydroxy acids) may be used in the compositions of the present invention at a suitable level to produce the desired result. In a preferred embodiment, they are present in a total amount of at least 0.1 wt-%, more preferably at least 0.25 wt-%, even more preferably at least 0.5 wt-%, and most preferably at least 1 wt-%, based on the ready to use concentration composition. In a preferred embodiment, they are present in a total amount of no greater than 10 wt-%, more preferably no greater than 5 wt-%, and even more preferably no greater than 3 wt-%, based on the ready to use composition.

The ratio of the total concentration of carboxylic acids (other than alpha- or beta-hydroxy acids) to the total concentration of the antiseptic component is preferably within a range of 10:1 to 1:100, and more preferably 2:1 to 1:10, on a weight basis.

Chelators. A chelating agent (i.e., chelator) is typically, an organic compound capable of multiple coordination sites with a metal ion in solution. Typically these chelating agents are polyanionic compounds and coordinate best with polyvalent metal ions. Exemplary chelating agents include, but are not limited to, ethylene diamine tetraacetic acid (EDTA) and salts thereof (e.g., EDTA(Na)₂, EDTA(Na)₄, EDTA(Ca), EDTA(K)₂), sodium acid pyrophosphate, acidic sodium hexametaphosphate, adipic acid, succinic acid, polyphosphoric acid, sodium acid pyrophosphate, sodium hexametaphosphate, acidified sodium hexametaphosphate, nitrilotris(methylenephosphonic acid), diethylenetriaminepentaacetic acid, 1-hydroxyethylene, 1,1-diphosphonic acid, and diethylenetriaminepenta-(methylenephosphonic acid). Certain carboxylic acids, particularly the alpha-hydroxy acids and beta-hydroxy acids, can also function as chelators, e.g., malic acid and tartaric acid. Also included are compounds highly specific toward ferrous or ferric ions such as siderophores and iron-bonding proteins such as lactoferrin and transferrin.

In certain preferred embodiments, the chelating agents useful in the compositions of the present invention include those selected from the group consisting of ethylenediaminetetraacetic acid and salts thereof, succinic acid, and mixtures thereof Preferably, either the free acid or the mono- or di-salt form of EDTA is used.

One or more chelating agents may be used in the compositions of the present invention at a suitable level to produce the desired result. In a preferred embodiment, they are present in a total amount of at least 0.01 wt-%, more preferably at least 0.05 wt-%, even more preferably at least 0.1 wt-%, and even more preferably at least 0.25 wt-%, based on the weight of the ready to use composition. Alternatively, in a preferred embodiment the chelators are present in a total amount of at least 300uM (micromolar), preferably at least 500uM, more preferably at least 1000uM and most preferably at least 2000uM based on the total weight/volume of composition even if it may comprise multiple phases. In a preferred embodiment, they are present in a total amount of no greater than 10 wt-%, more preferably no greater than 5 wt-%, and even more preferably no greater than 1 wt-%, based on the weight of the ready to use composition.

The ratio of the total concentration of chelating agents (other than alpha- or beta-hydroxy acids) to the total concentration of the antiseptic component is preferably within a range of 10:1 to 1:100, and more preferably 1:1 to 1:10, on a weight basis.

Phenolic Compounds. A phenolic compound enhancer is typically a compound having the following general structure (including at least one group bonded to the ring through an oxygen): 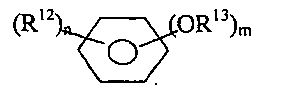 wherein: m is 0 to 3 (especially 1 to 3), n is 1 to 3 (especially 1 to 2), each R¹² independently is alkyl or alkenyl of up to 12 carbon atoms (especially up to 8 carbon atoms) optionally substituted with O in or on the chain (e.g., as a carbonyl group) or OH on the chain, and each R¹³ independently is H or alkyl or alkenyl of up to 8 carbon atoms (especially up to 6 carbon atoms) optionally substituted with O in or on the chain . (e.g., as a carbonyl group) or OH on the chain, but where R¹³ is H, n preferably is 1 or 2.

Examples of phenolic enhancers include, but are not limited to, butylated hydroxy anisole, e.g., 3(2)-tert-butyl-4-methoxyphenol (BHA), 2,6-di-tert-butyl-4-methylphenol (BHT), 3,5-di-tert-butyl-4-hydroxybenzylphenol, 2,6-di-tert-4-hexylphenol, 2,6-di-tert-4-octylphenol, 2,6-di-tert-4-decylphenol, 2,6-di-tert-butyl-4-ethylphenol, 2,6-di-tert-4-butylphenol, 2,5-di-tert-butylphenol, 3,5-di-tert-butylphenol, 4,6-di-tert-butyl-resorcinol, methyl paraben (4-hydroxybenzoic acid methyl ester), ethyl paraben, propyl paraben, butyl paraben, 2-phenoxyethanol, as well as combinations thereof. A preferred group of the phenolic compounds is the phenol species having the general structure shown above where R¹³ = H and where R¹² is alkyl or alkenyl of up to 8 carbon atoms, and n is 1, 2, or 3, especially where at least one R¹² is butyl and particularly tert-butyl, and especially the non-toxic members thereof. Some of the preferred phenolic enhancers are BHA, BHT, methyl paraben, ethyl paraben, propyl paraben, and butyl paraben as well as combinations of these.

One or more phenolic compounds may be used in the compositions of the present invention at a suitable level to produce the desired result. The concentrations of the phenolic compounds in medical-grade compositions may vary widely, but as little as 0.001 wt-%, based on the total weight of the composition, can be effective when the above-described esters are present within the above-noted ranges. In a preferred embodiment, they are present in a total amount of at least 0.01 wt-%, more preferably at least 0.10 wt-%, and even more preferably at least 0.25 wt-%, based on the ready to use composition. In a preferred embodiment, they are present in a total amount of no greater than 8 wt-%, more preferably no greater than 4 wt-%, and even more preferably no greater than 2 wt-%, based on the ready to use composition.

It is preferred that the ratio of the total phenolic concentration to the total concentration of the antiseptic component be within a range of 10:1 to 1:300, and more preferably within a range of 1:1 to 1:10, on a weight basis.

The above-noted concentrations of the phenolics are normally observed unless concentrated formulations for subsequent dilution are intended. On the other hand, the minimum concentration of the phenolics and the antiseptic components to provide an antimicrobial effect will vary with the' particular application.

Monohydroxy Alcohols. An additional enhancer class includes monohydroxy alcohol having 1-10 carbon atoms. This includes the lower (i.e., C1-C4) monohydroxy alcohols (e.g., methanol, ethanol, isopropanol, and butanol) as well as longer chain (i.e., C5-C10) monohydroxy alcohols (e.g., isobutanol, t-butanol, octanol, and decanol). In certain preferred embodiments, the alcohols useful in the compositions of the present invention are selected from the group consisting of methanol, ethanol, isopropyl alcohol, and mixtures thereof.

One or more alcohols may be used in the compositions of the present invention at a suitable level to produce the desired result. In a one embodiment, the short chain (i.e., C1-C4) alcohols are present in a total amount of at least 5 wt-%, even more preferably at least 10 wt-%, even more preferably at least 15 wt-%, and even more preferably at least 20 wt-%, based on the total weight of the ready to use composition. In a preferred embodiment, the (C1-C4)alcohols are present in a total amount of no greater than 50 wt-%, more preferably no greater than 40 wt-%, and even more preferably no greater than 30 wt-%, based on the total weight of the ready to use composition.

For certain applications, lower alcohols may not be preferred due to the strong odor and potential for stinging and irritation. This can occur especially at higher levels. In applications where stinging or burning is a concern, the concentration of (C1-C4)alcohols is preferably less than 20wt%, more preferably less than about 15wt%.

In preferred embodiments, longer chain (i.e., C5-C10) alcohols are present in a total amount of at least 0.1 wt-%, more preferably at least 0.25 wt-%, and even more preferably at least 0.5 wt-%, and most preferably at least 1.0wt-%, based on the ready to use composition. In a preferred embodiment, the (C5-C10)alcohols are present in a total amount of no greater than 10 wt-%, more preferably no greater than 5 wt-%, and even more preferably no greater than 2 wt-%, based on the total weight of the ready to use composition.

Ether glycols. An additional enhancer class includes ether glycol. Exemplary ether glycols include those of the formula:

R'-O-(CH₂CHR"O)ₙ(CH₂CHR"O)H

wherein R' = H, a (C1-C8)alkyl, a (C6-C12)aryl or a (C6-C12)aralkyl or (C6-C12)alkaryl; and each R" is independently = H, methyl, or ethyl; and n = 0-5, preferably 1-3. Examples include 2-phenoxyethanol, dipropylene glycol, triethylene glycol, the line of products available under the trade designation DOWANOL DB (di(ethylene glycol) butyl ether), DOWANOL DPM (di(propylene glycol)monomethyl ether), and DOWANOL TPnB (tri(propylene glycol) monobutyl ether), as well as many others available from Dow Chemical, Midland MI.

One or more ether glycols may be used in the compositions of the present invention at a suitable level to produce the desired result. In a preferred embodiment, they are present in a total amount of at least 0.01 wt-%, based on the total weight of the ready to use composition. In a preferred embodiment, they are present in a total amount of no greater than 20 wt-%, based on the total weight of the ready to use composition.

### Surfactants

Compositions of the present invention can include one or more surfactants to emulsify the composition and to help the composition wet the surface and/or to aid in contacting the microorganisms. As used herein the term "surfactant" means an amphiphile (a molecule possessing both polar and nonpolar regions which are covalently bound) capable of reducing the surface tension of water and/or the interfacial tension between water and an immiscible liquid. The term is meant to include soaps, detergents, emulsifiers, surface active agents and the like. The surfactant can be cationic, anionic, nonionic, or amphoteric. This includes a wide variety of conventional surfactants. Combinations of surfactants can be used if desired.

Certain ethoxylated surfactants may reduce or eliminate the antimicrobial efficacy of the antiseptic component. The exact mechanism of this is not known and not all ethoxylated surfactants display this negative effect. For example, poloxamer (polyethylene oxide/polypropylene oxide) surfactants have been shown to be compatible with some antiseptic components, but ethoxylated sorbitan fatty acid esters such as those sold under the trade name TWEEN by ICI have not been compatible and may even be useful in neutralizing the antiseptic in microbiological assays. Furthermore, certain anionic surfactants may not be compatible with the cationic antiseptics optionally present in the compositions of this invention. It should be noted that these are broad generalizations and the activity could be formulation dependent. One skilled in the art can easily determine compatibility of a surfactant by making the formulation and testing for antimicrobial activity as described in the Examples Section.

It should be noted that certain antiseptics are amphiphiles and may be surface active. For example, the fatty acid antiseptics described herein are surface active. For those compositions that include both an amphiphilic antiseptic and a surfactant, the surfactant is a component separate from the amphiphilic antiseptic.

Preferred surfactants are those that have an HLB (i.e., hydrophile to lipophile balance) of at least 4 and more preferably at least 8. Even more preferred surfactants have an HLB of at least 12. Most preferred surfactants have an HLB of at least 15.

Examples of the various classes of surfactants are described below. In certain preferred embodiments, the surfactants useful in the compositions of the present invention are selected from the group consisting of sulfonates, sulfates, phosphonates, phosphates, poloxamer (polyethylene oxide/polypropylene oxide block copolymers), cationic surfactants, and mixtures thereof. In certain more preferred embodiments incorporating non-ionic or anionic antiseptics, the surfactants useful in the compositions of the present invention are selected from the group consisting of sulfonates, sulfates, phosphates, and mixtures thereof. Cationic, amphoteric, and non-ionic surfactants and in particular the ethylene oxide/propylene oxide surfactants such as poloxamers are particularly preferred for use if other cationic components are present (e.g., an optional cationic antiseptic such as those described in "Cationic Antiseptic Compositions and Methods of Use," U.S. Serial No. 10/936,135, filed September 7, 2004.

One or more surfactants may be used in the compositions of the present invention at a suitable level to produce the desired result. In many instances, the compositions of the present invention are intended to be left on tissue in the desired application. For those surfactants that can be irritating to tissue, the surfactants are preferably present in low concentrations, i.e. present in a total amount of no greater than 10 wt-%. In a more preferred embodiment, they are present in an amount no greater than 5 wt-%, and even more preferably no greater than 3 wt-%, based on the total weight of the ready to use composition. In a preferred embodiment, they are present in a total amount of at least 0.01 wt-%, preferably at least 0.1wt-%, more preferably at least 0.5 wt-%, and even more preferably at least 1.0 wt-%, based on the total weight of the ready to use composition. The ratio of the total concentration of surfactant to the total concentration of the antiseptic is preferably within a range of 5:1 to 1:100, more preferably 3:1 to 1:10, and most preferably 2:1 to 1:3, on a weight basis.

Cationic Surfactants. Exemplary cationic surfactants include, but are not limited to, salts of optionally polyoxyalkylenated primary, secondary, or tertiary fatty amines; quaternary ammonium salts such as tetraalkylammonium, alkylamidoalkyltrialkylammonium, trialkylbenzylammonium, trialkylhydroxyalkylammonium, or alkylpyridinium having compatible anionic couterions such as halides (preferably chlorides or bromides) or alkyl sulfates such as methosulfate and ethosulfate; imidazoline derivatives; amine oxides of a cationic nature (e.g., at an acidic pH), and mixtures thereof.

In certain preferred embodiments, the cationic surfactants useful in the compositions of the present invention are selected from the group consisting of tetralkyl ammonium, trialkylbenzylammonium, and alkylpyridinium halides or alkyl sulfates such as metnosulfate and etnosulfate as well as other anionic counterions, and mixtures thereof.

Also particularly preferred are amine oxide surfactants including alkyl and alkylamidoalkyldialkylamine oxides of the following formula:

(R¹⁴)₃-N→O

wherein R¹⁴ is a (C1-C30)alkyl group (preferably a (C1-C14)alkyl group) or a (C6-C18)aralklyl or alkaryl group, wherein any of these groups can be optionally substituted in or on the chain by N-, O-, or S-containing groups such as amide, ester, hydroxyl, and the like. Each R¹⁴ may be the same or different provided at least one R¹⁴ group includes at least eight carbons. Optionally, the R¹⁴ groups can be joined to form a heterocyclic ring with the nitrogen to form surfactants such as amine oxides of alkyl morpholine, alkyl piperazine, and the like. Preferably two R¹⁴ groups are methyl and one R¹⁴ group is a (C12-C16)alkyl or alkylamidopropyl group. Examples of amine oxide surfactants include those commercially available under the trade designations AMMONYX LO, LMDO, and CO, which are lauryldimethylamine oxide, laurylamidopropyldimethylamine oxide, and cetyl amine oxide, all from Stepan Company ofNorthfield, IL.

Anionic Surfactants. Exemplary anionic surfactants include, but are not limited to, sarcosinates, glutamates, alkyl sulfates, sodium or potassium alkyleth sulfates, ammonium alkyleth sulfates, ammonium laureth-n-sulfates, laureth-n-sulfates, isethionates, alkyl or aralkyl glycerylether sulfonates, alkyl or aralkyl sulfosuccinates, alkylglyceryl ether sulfonates, alkyl phosphates, aralkyl phosphates, alkylphosphonates, and aralkylphosphonates. These anionic surfactants may have a mono- or divalent metal or organic ammonium counterion In certain preferred embodiments, the anionic surfactants useful in the compositions of the present invention are selected from the group consisting of:
1. *Sulfonates and Sulfates.* Suitable anionic surfactants include sulfonates and sulfates such as alkyl sulfates, alkylether sulfates, alkyl sulfonates, alkylether sulfonates, alkylbenzene sufonates, alkylbenzene ether sulfates, alkylsulfoacetates, secondary alkane sulfonates, secondary alkylsulfates, and the like. Many of these can be represented by the formulas:

   R¹⁴-(OCH₂CH₂)ₙ(OCH(CH₃)CH₂)ₚ-(Ph)ₐ-(OCH₂CH₂)ₘ-(O)_{b}-SO₃⁻M⁺

   and

   R¹⁴ -CH[SO₃- M⁺]- R¹⁵

   wherein: a and b = 0 or 1; n, p, and m = 0-100 (preferably 0-20, and more preferably 0-10); R¹⁴ is defined as above provided at least one R¹⁴ or R¹⁵ is at least C8; R¹⁵ is a (C1-C12)alkyl group (saturated straight, branched, or cyclic group) that may be optionally substituted by N, O, or S atoms or hydroxyl, carboxyl, amide, or amine groups; Ph =' phenyl; and M is a cationic counterion such as H, Na, K, Li, ammonium, or a protonated tertiary amine such as triethanolamine or a quaternary ammonium group.
   In the formula above, the ethylene oxide groups (i.e., the "n" and "m" groups) and propylene oxide groups (i.e., the "p" groups) can occur in reverse order as well as in a random, sequential, or block arrangement. Preferably for this class, R¹⁴ includes an alkylamide group such as R¹⁶-C(O)N(CH₃)CH₂CH₂- as well as ester groups such as - OC(O)-CH₂- wherein R¹⁶ is a (C8-C22)alkyl group (branched, straight, or cyclic group). Examples include, but are not limited to: alkyl ether sulfonates such as lauryl ether sulfates such as POLYSTEP B12 (n = 3-4, M = sodium) and B22 (n =12, M = ammonium) available from Stepan Company, Northfield, IL and sodium methyl taurate (available under the trade designation NIKKOL CMT30 from Nikko Chemicals Co., Tokyo, Japan); secondary alkane sulfonates such as Hostapur SAS which is a Sodium (C14-C17)secondary alkane sulfonates (alpha-olefin sulfonates) available from Clariant Corp., Charlotte, NC; methyl-2-sulfoalkyl esters such as sodium methyl-2-sulfo(C12-16)ester and disodium 2-sulfo(C12-C16)fatty acid available from Stepan Company under the trade designation ALPHASTEP PC-48; alkylsulfoacetates and alkylsulfosuccinates available as sodium laurylsulfoacetate (under the trade designation LANTHANOL LAL) and disodiumlaurethsulfosuccinate (STEPANMILD SL3), both from Stepan Company; alkylsulfates such as ammoniumlauryl sulfate commercially available under the trade designation STEPANOL AM from Stepan Company; dialkylsulfosuccinates such as dioctylsodiumsulfosuccinate available as Aerosol OT from Cytec Industries.
*2. Phosphates and Phosphonates.* Suitable anionic surfactants also include phosphates such as alkyl phosphates, alkylether phosphates, aralkylphosphates, and aralkylether phosphates. Many may be represented by the formula:

   [R¹⁴-(Ph)ₐ-O(CH₂CH₂O)ₙ(CH₂CH(CH₃)O)ₚ]_{q}-P(O)(O⁻M⁺]ᵣ
wherein: Ph, R¹⁴, a, n, p, and M are defined above; r is 0-2; and q = 1-3; with the proviso that when q = 1, r = 2, and when q = 2, r = 1, and when q = 3, r = 0. As above, the ethylene oxide groups (i.e., the "n" groups) and propylene oxide groups (i.e., the "p" groups) can occur in reverse order as well as in a random, sequential, or block arrangement. Examples include a mixture of mono-, di- and tri-(alkyltetraglycolether)-o-phosphoric acid esters generally referred to as trilaureth-4-phosphate commercially available under the trade designation HOSTAPHAT 340KL from Clariant Corp., as well as PPG-5 ceteth 10 phosphate available under the trade designation CRODAPHOS SG from Croda Inc., Parsipanny, NJ, and mixtures thereof.

Amphoteric Surfactants. Surfactants of the amphoteric type include surfactants having tertiary amine groups, which may be protonated, as well as quaternary amine containing zwitterionic surfactants. Those that have been particularly useful include:
*1. Ammonium Carboxylate Amphoterics.* This class of surfactants can be represented by the following formula:

   R¹⁷C(O)-NH)ₐR¹⁸N⁺(R¹⁹)₂-R²⁰-COO⁻

   wherein: a = 0 or 1; R¹⁷ is a (C7-C21)alkyl group (saturated straight, branched, or cyclic group), a (C6-C22)aryl group, or a (C6-C22)aralkyl or alkaryl group (saturated straight, branched, or cyclic alkyl group), wherein R¹⁷ may be optionally substituted with one or more N, O, or S atoms, or one or more hydroxyl, carboxyl, amide, or amine groups; R¹⁹ is H or a (C1-C8)alkyl group (saturated straight, branched, or cyclic group),
   wherein R¹⁹ may be optionally substituted with one or more N, O, or S atoms, or one or more hydroxyl, carboxyl, amine groups, a (C6-C9)aryl group, or a (C6-C9)aralkyl or alkaryl group; and R¹⁸ and R²⁰ are each independently a (C₁-C₁₀)alkylene group that may be the same or different and may be optionally substituted with one or more N, O, or S atoms, or one or more hydroxyl or amine groups.
   More preferably, in the formula above, R¹⁷ is a (C1-C18)alkyl group, R¹⁹ is a (C1-C2)alkyl group preferably substituted with a methyl or benzyl group and most preferably with a methyl group. When R¹⁹ is H it is understood that the surfactant at higher pH values could exist as a tertiary amine with a cationic counterion such as Na, K, Li, or a quaternary amine group.
   Examples of such amphoteric surfactants include, but are not limited to: certain betaines such as cocobetaine and cocamidopropyl betaine (commercially available under the trade designations MACKAM CB-35 and MACKAM L from McIntyre Group Ltd., University Park, IL); monoacetates such as sodium lauroamphoacetate; diacetates such as disodium lauroamphoacetate; amino- and alkylamino-propionates such as lauraminopropionic acid (commercially available under the trade designations MACKAM 1 L, MACKAM 2L, and MACKAM 151L, respectively, from McIntyre Group Ltd.).
*2. Ammonium Sulfonate Amphoterics.* This class of amphoteric surfactants are often referred to as "sultaines" or "sulfobetaines" and can be represented by the following formula

   R¹⁷-(C(O)-NH)ₐ-R¹⁸-N⁺(R¹⁹)₂-R²⁰-SO₃-
wherein R¹⁷-R²⁰ and "a" are defined above. Examples include cocamidopropylhydroxysultaine (commercially available as MACKAM 50-SB from McIntyre Group Ltd.). The sulfoamphoterics may be preferred over the carboxylate amphoterics since the sulfonate group will remain ionized at much lower pH values.

Nonionic Surfactants. Exemplary nonionic surfactants include, but are not limited to, alkyl glucosides, alkyl polyglucosides, polyhydroxy fatty acid amides, sucrose esters, esters of fatty acids and polyhydric alcohols, fatty acid alkanolamides, ethoxylated fatty acids, ethoxylated aliphatic acids, ethoxylated fatty alcohols (e:g., octyl phenoxy polyethoxyethanol available under the trade name TRITON X-100 and nonyl phenoxy poly(ethyleneoxy) ethanol available under the trade name NOWDET P-40, both from Sigma, St. Louis, MO), ethoxylated and/or propoxylated aliphatic alcohols (e.g., that available under the trade name Brij from ICI), ethoxylated glycerides, ethoxylated/propoxylate block copolymers such as Pluronic and Tetronic from BASF, ethoxylated cyclic ether adducts, ethoxylated amide and imidazoline adducts, ethoxylated amine adducts, ethoxylated mercaptan adducts, ethoxylated condensates with alkyl phenols, ethoxylated nitrogen-based hydrophobes, ethoxylated polyoxypropylenes, polymeric silicones, fluorinated surfactants (e.g., those available under the trade names FLUORAD-FS 300 from 3M Company, St. Paul, MN, and ZONYL from Dupont de Nemours Co., Wilmington, DE), and polymerizable (reactive) surfactants (e.g., SAM 211 (alkylene polyalkoxy sulfate) surfactant available under the trade name MAZON from PPG Industries, Inc., Pittsburgh, PA). In certain preferred embodiments, the nonionic surfactants useful in the compositions of the present invention are selected from the group consisting of Poloxamers such as PLURONIC from BASF, sorbitan fatty acid esters, and mixtures thereof.

### Hvdrophilic Component

Compositions of the present invention include a hydrophilic or water-soluble component to help solubilize and/or physically stabilize the antiseptic and/or enhancer component in the composition and/or to enhance the antimicrobial efficacy and/or the speed of antimicrobial efficacy. Incorporation of a sufficient amount of hydrophilic component in hydrophobic ointments can increase the antimicrobial activity both in terms of speed and extent of kill. While not intended to be bound by theory, the incorporation of the hydrophilic component may allow more antiseptic to be available at the surface or to more rapidly diffuse to the surface of the ointment during use. Certain compositions may be solutions, emulsions (one liquid/gel/paste dispersed in another liquid/gel/paste), or dispersions (solid in liquid/paste/gel) or combinations thereof.

In general, the ratio of total hydrophilic component to total hydrophobic component (water insoluble ingredients) is at least 5:95 wt/wt, preferably at least 10:90 wt/wt, more preferably at least 15:85 wt/wt and most preferably at least 20:80 wt/wt. Levels as high as 30:70, 40:60, and 50:50 wt/wt of total hydrophilic component to total hydrophobic component (water insoluble ingredients) or higher may be appropriate for certain compositions.

A hydrophilic material is typically a compound that has a solubility in water of at least 7 wt-%, preferably at least 10 wt-%, more preferably at least 20 wt-%, even more preferably at least 25 wt-%, and even more preferably at least 40 wt-%, at 23°C. Most preferably, a hydrophilic component is infinitely miscible with water at 23°C.

The hydrophilic components include water, polyhydric alcohols, lower alkyl ethers (i.e., having a sufficiently small number of carbon atoms to meet the solubility limit above), N-methylpyrrolidone, alkyl esters (i.e., having a sufficiently small number of carbon atoms to meet the solubility limit above), and the lower monohydroxy alcohols discussed above as enhancers, as well as combinations thereof. Thus, a lower monohydroxy alcohol can function as both a hydrophilic compound and an enhancer. Preferably, the hydrophilic components include polyhydric alcohols, lower alkyl ethers, and short chain esters. More preferably, the hydrophilic components include polyhydric alcohols.

Suitable polyhydric alcohols (i.e., organic compounds having more than one hydroxyl group) have a molecular weight of less than 500, preferably less than 400, and more preferably less than 200. Examples of polyhydric alcohols include, but are not limited to, glycerol, propylene glycol, dipropylene glycol, tripropylene glycol, polypropylene glycol, diethylene glycol, triethylene glycol, polyethylene glycol, pentaerythritol, trimethylolpropane, trimethylolethane, trimethylolbutane, sorbitol, mannitol, xylitol, pantothenol, ethylene glycol adducts of polyhydric alcohol, propylene oxide adducts of polyhydric alcohol, 1,3-butanediol, dipropylene glycol, diglycerine, polyglycerine, erythritol, sorbitan, sugars (e.g., sucrose, glucose, fructose, mannose, xylose, saccharose, trehalose), sugar alcohols, and the like. Certain preferred polyhydric alcohols include glycols (i.e., those containing two hydroxyl groups) including glycerin and propylene glycol. Certain other preferred polyhydric alcohols include xylitol, mannitol, sorbitol, sucrose and polyglycerin.

Ethers include materials such as dimethylisosorbide, polyethylene glycol and methoxypolyethylene glycols, block and random copolymers of ethylene oxide and propylene oxide, and laureth-4. Alkyl esters include triacetin, methyl acetate, esters of polyethoxylated glycols, and combinations thereof.

In certain preferred embodiments, the hydrophilic components useful in the compositions of the present invention include those selected from the group consisting of glycols, and in particular glycerin and propylene glycol, and mixtures thereof.

If there are components in the composition which may esterify with hydroxylfunctional hydrophilic components, conditions are selected to minimize this occurrence. For example, the components are not heated together for extended periods of time, and/or the pH is close to neutral if possible, etc.

One or more hydrophilic materials may be used in the'compositions of the present invention at a suitable level to produce the desired result. In certain preferred embodiments that also include the hydrophobic component as the primary component (i.e., the component used in the greatest amount and referred to as a "vehicle"), the hydrophilic component is present in a total amount of at least 1 wt-%, more preferably at least 4 wt-%, and even more preferably at least 8 wt-%, based on the weight of the ready to use composition. In certain embodiments, for example when faster rate of kill is desired, higher levels of hydrophilic component may be employed. In these cases the hydrophilic component is present in a total amount of at least 10% by weight, more preferably at least 20% by weight and most preferably at least 25% by weight. In a preferred embodiment, the hydrophilic component is present in a total amount of no greater than 70 wt-%, more preferably no greater than 60 wt-%, and even more preferably no greater than 50 wt-%, based on the ready to use composition. When the hydrophilic component is present in the greatest amount it is referred to as a "vehicle." When a slower release of the antiseptic is desired the hydrophilic component is present in an amount no greater than about 30% by weight.

For certain applications it may be desirable to formulate these antiseptics in compositions comprising a hydrophilic component vehicle that is thickened with soluble; swellable or insoluble (e.g., insoluble) organic polymeric thickeners or inorganic thickeners such as silica, fumed silica, precipitated silica, silica aerogel and carbon black, and the like; other particle fillers such as calcium carbonate, magnesium carbonate, kaolin, talc, titanium dioxide, aluminum silicate, diatomaceous earth, ferric oxide and zinc oxide, clays, and the like; ceramic microspheres or glass microbubbles; ceramic microspheres suc as those available under the tradenames "ZEOSPHERES" or "Z-LIGHT" from 3M. The above fillers can be used alone or in combination.

If water is used as the hydrophilic component in certain embodiments, it is present in an amount of less than 20 wt %, preferably less than 10 wt-%, more preferably less than 5 wt-%, and even more preferably less than 2 wt-%, based on the ready to use composition. This helps the chemical stability of the compositions and may reduce irritation. For certain other embodiments, water can be used in a much greater amount, and can even be the primary component, as long as the composition is highly viscous. Preferably, such highly viscous compositions have a viscosity of at least 500 centipoise (cps), more preferably at least 1,000 cps, even more preferably at least 10,000 cps, even more preferably at least 20,000 cps, even more preferably at least 50,000 cps, even more preferably at least 75,000 cps, even more preferably at least 100,000 cps, and even more preferably at least 250,000 cps (and even as high as about 500,000 cps, 1,000,000 cps, or more). The viscosity can be measured as described below in the Viscosity Test. Most preferred compositions meet these viscosity values even after heating to 32°C, preferably 35°C or as high as 37°C to ensure when in contact with mammalian tissue the compositions remain substantive.

### Hydrophobic Component

The compositions of the present invention also include one or more hydrophobic materials. A hydrophobic material is typically an organic compound, which at 23°C is a liquid, gelatinous, semisolid or solid and has a solubility in water of less than 5% by weight, preferably less than 1% by weight, more preferably less than 0.5% by weight, and most preferably less than 0.1% by weight. These materials include compounds typically considered emollients in the cosmetic art.

Examples of general emollients include, but are not limited to, short chain (i.e, C1-C6) alkyl or (C6-C12)aryl esters of long (i.e., C8-C36) straight or branched chain alkyl or alkenyl alcohols or acids and polyethoxylated derivatives of the alcohols; short chain (i.e., C1-C6) alkyl or (C6-C12)aryl esters of (C4-C12)diacids or (C4-C12)diols optionally substituted in available positions by -OH; (C2-C18)alkyl or (C6-C12)aryl esters of glycerol, pentaerythritol, ethylene glycol, propylene glycol, as well as polyethoxylated derivatives of these; (C12-C22)alkyl esters or (C12-C22)ethers of polypropylene glycol; (C12-C22)alkyl esters or (C12-C22)ethers of polypropylene glycol/polyethylene glycol copolymer; and polyether polysiloxane copolymers.

Additional examples of hydrophobic components include cyclic dimethicones including volatile cyclic silicones such as D3 and D4, polydialkylsiloxanes, polyaryl/alkylsiloxanes, silicone copolyols, long chain (i.e., C8-C36) alkyl and alkenyl esters of long (i.e., C8-C18) straight or branched chain alkyl or alkenyl alcohols or acids, long chain (i.e., C8-C36) alkyl and alkenyl amides of long straight or branched chain (i.e., C8-C36) alkyl or alkenyl amines or acids; hydrocarbons including straight and branched chain alkanes and alkenes such as isoparafins (e.g., isooctane, isododecane, isooctadecane, etc.), squalene, and mineral oil, polysiloxane polyalkylene copolymers, dialkoxy dimethyl polysiloxanes; (C12-C22)alkyl and (C12-C22)alkenyl alcohols, and petroleum derived alkanes such as isoparafins, petrolatum, petrolatum USP, as well as refined natural oils (especially NF or USP grades) such as olive oil NF, cotton seed oil, peanut oil, corn oil, seasame oil, safflower oil, soybean oil, and the like, and blends thereof.

In certain preferred embodiments, the hydrophobic components useful in the compositions of the present invention include those selected from the group consisting of petrolatum USP and short chain (i.e., C1-C6) alkyl or (C6-C12)aryl esters of long (i.e., C8-C36) .straight or branched chain alkyl or alkenyl alcohols or acids and polyethoxylated derivatives of the alcohols; short chain (i.e., C1-C6) alkyl or (C6-C12)aryl esters of (C4-C12)diacids or (C4-C12)diols optionally substituted in available positions by-OH (such as diisopropyladipate, diisopropylsebacate); (C1-C9)alkyl or (C6-C12)aryl esters of glycerol, pentaerythritol, ethylene glycol, propylene glycol (such as glyceryl tricaprylate/caprate); and mixtures thereof. For certain particularly preferred embodiments, the hydrophobic component is petrolatum.

One or more hydrophobic materials may be used in the compositions of the present invention at a suitable level to produce the desired result. The hydrophobic component forms the greatest portion of the composition, i.e. is present in a total amount of at least 50 wt%, more preferably at least 60 wt-%, and even more preferably at least 70 wt-%, based on the ready to use composition. In a preferred embodiment, the hydrophobic component is present in a total amount of no greater than 99 wt-%, more preferably no greater than 95 wt-%, and even more preferably no greater than 92 wt-%, based on the ready to use composition. When the hydrophobic component is present in the greatest amount it is referred to as a "vehicle." In those formulations where the hydrophobic component(s) and the hydrophilic component(s) are present at the same concentrations, the continuous phase is considered the "vehicle".

### Optional Additives

Compositions of the present invention may additionally employ adjunct components conventionally found in pharmaceutical compositions in their art-established fashion and at their art-established levels. Thus, for example, the compositions may contain additional compatible pharmaceutically active materials for combination therapy (such as supplementary antimicrobials, anti-parasitic agents, antipruritics, astringents, local anaesthetics, steroids, non-steorodial antinflammatory agents, or other anti-inflammatory agents), or may contain materials useful in physically formulating various dosage forms of the present invention, such as excipients, dyes, perfumes, fragrances, lubricants, thickening agents, stabilizers, skin penetration enhancers, preservatives, or antioxidants.

It will be appreciated by the skilled artisan that the levels or ranges selected for the required or optional components described herein will depend upon whether one is formulating a composition for direct use, or a concentrate for dilution prior to use, as well as the specific component selected, the ultimate end-use of the composition, and other factors well known to the skilled artisan.

It will also be appreciated that additional antiseptics, disinfectants, or antibiotics may be included and are contemplated. These include, for example, addition of metals such as silver, copper, zinc; iodine and iodophors: "azole" antifungal agents including clortrimazole, miconazole, econazole, ketoconazole, and salts thereof; and the like. Antibiotics such as neomycin sulfate, bacitracin, mupirocin, tetracycline, polymixin, and the like, also may be included. Preferred compositions, however, are free of antibiotics due to the chance of resistance formation.'

### Formulations and Methods of Preparation

Many of the compositions of the present invention demonstrate a broad spectrum of antimicrobial activity and thus are generally not terminally sterilized but if necessary may be sterilized by a variety of industry standard techniques. For example, it may be preferred to sterilize the compositions in their final packaged form using electron beam. It may also be possible to sterilize the sample by gamma radiation or heat. Other forms of sterilization may be acceptable. It may also be suitable to include preservatives in the formulation-to prevent growth of certain organisms. Suitable preservatives include industry standard compounds such as parabens (methyl, ethyl, propyl, isopropyl, isobutyl, etc), 2 bromo-2 nitro-1,3, diol; 5 bromo-5-nitro-1,3 dioxane, chlorbutanol, diazolidinyl urea; iodopropylnyl butylcarbamate, phenoxyethanol, halogenated cresols, methylchloroisothiazolinone and the like, as well as combinations of these compounds.

The compositions of the present invention preferably adhere well to mammalian tissue (e.g, skin, mucosal tissue, and wounds), in order to deliver the antimicrobial to the intended site over a prolonged period even in the presence of perspiration, drainage (e.g., mucosal secretions), or mild lavage. The compositions are typically non-aqueous, although high viscosity compositions can include a large amount of water. The component in the greatest amount (i.e., the vehicle) in the formulations of the invention may be any conventional vehicle commonly used for topical treatment of human or animal skin. The formulations are typically selected from one of the following five types: (1) formulations with a hydrophobic vehicle (i.e., the hydrophobic component, which can include one or more hydrophobic compounds, present in the greatest amount) which may be anhydrous, nearly anhydrous or further comprise a aqueous phase; (2) formulations based on water in oil emulsions in which the water insoluble continuous "oil" phase is comprised of one or more hydrophobic components; 3) formulations with a hydrophilic vehicle (i.e., the hydrophilic component, which can include one or more hydrophilic compounds, is present in the greatest amount) which may be anhydrous, nearly anhydrous or further comprise a aqueous phase; (4) highly viscous water-based formulations formulations which may be solutions or oil in water emulsions; and 5) neat compositions which are essentially free of a hydrophobic or hydrophilic vehicle component comprising antiseptic, optionally an enhancer, and further optionally a surfactant. In this latter case the compositions may optionally be dissolved in a volatile carrier solvent for delivery to the intended treatment site or may be delivered to the site as a dry powder, liquid, or semi-solid composition. The different types of compositions are discussed further below.
(1) Anhydrous or Nearly Anhydrous Formulations with a Hydrophobic Vehicle: In certain preferred embodiments of the present invention, the compositions include an antiseptic component in a hydrophobic vehicle optionally in combination with surfactant(s), an enhancer component, and a small amount of a hydrophilic component. In most instances the enhancers are not soluble in the hydrophobic component at room temperature although they may be at elevated temperatures. The hydrophilic component is generally present in a sufficient amount to stabilize (and perhaps to solubilize) the enhancer(s) in the composition. For example, when formulating with organic acid enhancers or certain solid surfactants or certain antiseptics in petrolatum many antiseptics, enhancers, and surfactants will dissolve into the petrolatum at temperatures above 85°C; however, upon cooling, the antiseptic, enhancer and/or surfactant crystals or precipitates back out of solution making it difficult to produce a uniform formulation. If at least 0.1 wt-% and preferably at least 1.0wit-%, more preferably at least 5%, and most preferably at least 10 wt-% of a hydrophilic compound (e.g., a glycol) is added a stable formulation can be obtained. It is believed that these formulations produce an emulsion in which the enhancer and/or surfactant is dissolved, emulsified, or dispersed in the hydrophilic component which is emulsified into the hydrophobic component(s). These compositions are stable upon cooling and centrifuging.
   The hydrophilic component also helps to stabilize many of the surfactants used in preferred formulations. For example, dioctylsulfosuccinate sodium salt (DOSS) dissolves in glycerin at elevated temperatures and helps keep the DOSS physically stable in the composition. Furthermore, it is believed that incorporation of the hydrophilic component in the formulation improves the antimicrobial activity. The mechanism for this is unknown; however, it may speed the release of the enhancer component and/or the antiseptic component.
   The water content of these formulations is preferably less than 20 wt-%, more preferably less than 10 wt-%, and even more preferably less than 5 wt-%, and most preferably less than 2 wt-%, in order to minimize chemical degradation of antiseptics present as well as to reduce concerns with microbial contamination in the composition during storage, and to reduce irritation of the tissue to which it is applied.
   These formulations can be manufactured with relative ease. The following description assumes all components are present in order to describe their manufacture. It is understood, however, that certain compositions may not contain one or more of these components.' In one method the compositions are manufactured by first heating the hydrophobic component to 85°C, adding in the surfactant, hydrophilic component, and optional enhancer component, cooling to 65°C, and adding the antiseptic component which may be above its melting point. Alternatively, the enhancer. component, if used, can be predissolved in the hydrophilic component (optionally along with the surfactant) and added to the hydrophobic component either before or after addition of the antiseptic component. If either the antiseptic component or the hydrophobic component is solid at room temperature, this is done at the minimum temperature necessary to ensure dissolution and uniformity of the composition. Exposure of ester-containing antiseptics or excipients to enhancers or other components comprising either acid or hydroxyl groups at elevated temperatures for extended periods of time should be avoided to prevent transesterification reactions. There are exceptions, for example, when heating lower purity fatty acid esters in combination with glycol hydrophilic components to produce the monoesters of higher purity.
   Thus, the present invention provides methods of manufacture. One method involves: combining the hydrophobic vehicle and the hydrophilic component with mixing to form a mixture; optionally heating the hydrophobic vehicle to a temperature sufficient to form a pourable liquid (which for many hydrophobic vehicles this is above its melting point) before or after combining it with the hydrophilic component; adding the antiseptic component to the mixture; and cooling the mixture before or after adding the antiseptic component.
   One preferred method involves: dissolving at least a portion of the enhancer component in the hydrophilic component; combining the hydrophobic vehicle and the hydrophilic component with the enhancer component dissolved therein with mixing to form a mixture; optionally heating the hydrophobic vehicle to a temperature sufficient to form a pourable liquid (which for many hydrophobic vehicles this is above its melting point) before or after combining it with the hydrophilic component and enhancer component; adding the antiseptic component to the mixture; and cooling the mixture before or after adding the antiseptic component.
   The hydrophilic component may or may not be present in the formulations that include a hydrophobic vehicle. Thus, another preferred method of manufacture involves: optionally heating the hydrophobic vehicle to a temperature sufficient to form a pourable liquid (which for many hydrophobic vehicles is above its melting point) before or after combining it with the optional enhancer component; adding the antiseptic component to the mixture with mixing; and cooling the mixture before or after adding the antiseptic component.
   Surprisingly, it has been found that these compositions are significantly less irritating than formulations using hydrophilic vehicles. In blind human trials participants were asked to instill 0.5 gram (g) of ointments based on hydrophobic components (e.g., petrolatum) that include an AHA enhancer, surfactant, and 10wt-% hydrophilic component (e.g., glycerin) as well as ointments based on hydrophilic components (e.g., PEG 400) using the same enhancer and surfactant. The ointments with the hydrophobic vehicle were preferred by 100% of the participants.
   Most preferably, the formulations intended for use on skin, anterior nares, or where drainage would be a concern are essentially gelatinous at room temperature, having a significant yield point such that they do not flow readily at temperatures below 35°C. The viscosity is measured using the viscosity test described herein. Certain gelatinous vehicles may also have a characteristic temperature at which they "melt" or begin to dramatically lose viscosity. Preferably this is higher than body temperature also to ensure that excess drainage of the composition of the treatment site does not occur. Therefore, the melting point of the composition is preferably greater than 32°C, more preferably greater than 35°C, and even more preferably greater than about 37°C. The melting point is taken as the lowest temperature at which the viscosity becomes dramatically less or is equal to or less than 100,000 cps.
   Alternatively, formulations could be considered which gel or thicken when warmed to body temperature. For example, aqueous compositions based on Pluronic F127 (e.g., greater than about 17% by weight), as well as other Poloxamers of similar structure, are relatively low viscosity at 4°C but when warmed to body temperature become very viscous. In these applications, the viscosity should be measured at 35°C. Similarly the viscosity and/or melt temperature can be enhanced by either incorporating a crystalline or semicrystalline emulsifier and/or hydrophobic carrier such as a higher melting petrolatum, addition of an insoluble filler/thixotrope, or by addition of a polymeric thickener (e.g., a polyethylene wax in a petrolatum vehicle). Polymeric thickeners may be linear, branched, or slightly crosslinked. It is important for comfort that the formulations are relatively soft and that they spread easily to allow easy application, especially over a wound, rash, or infected area or in the anterior nares. A particularly preferred vehicle for use on skin, in the anterior nares, or in other areas where high viscosity is desirable is white petrolatum USP having a melting point greater than 40°C.
(2) Water in Oil Emulsions: Antiseptic components of this invention can be formulated into water-in-oil emulsions in combination with enhancer(s) and surfactant(s). These compositions comprise at least 50% by weight oil phase. As used herein the oil phase is comprised of all components which are either not soluble in water or preferentially soluble in the oil(s) present at 23°C. According to one method of preparing these emulsions the hydrophobic component (oil) is mixed in Container A along with any emulsifier(s) optionally including polymeric emulsifiers and heated to a temperature sufficient to ensure a homogenous composition and subsequent stable emulsion. The temperature is typically raised to at least 60°C, preferably to at least 80°C and more preferably to 100°C or more. In a separate Container B, the hydrophilic ingredients are mixed, including one or more of the following: water, hydrophilic component, enhancer(s), surfactant(s), and acids/bases to adjust the pH of the final composition. The contents of container B are heated to a temperature sufficient to ensure a stable final emulsion composition without significantly degrading any of the components, typically greater than 40°C, preferably greater than 50°C and more preferably to greater than 60°C. While hot, container B is added to container A using a high shear mixer. The composition may be continuously mixed until cool (T<40°C) or it can be allowed to sit as long as the contents remain uniformly mixed. If the antiseptic is heat sensitive, it is added with mixing during the cooling down period. If it is not heat sensitive, it may be added to either container A or container B. The viscosity of these compositions may be adjusted by altering the levels of emulsifier; changing the ratio of water to oil phase; selection of the oil phase (e.g., select an oil (hydrophobic component) which is more or less viscous); incorporation of a polymeric or particulate thickener, etc.
(3) Hydrophilic Vehicle: Antiseptic components of this invention can be formulated into a hydrophilic component such as that based on the hydrophilic compounds discussed above optionally in combination with the enhancer(s) and surfactant(s). Particularly preferred are polyethylene glycols (PEGs), glycols, and combinations thereof, including blends of different molecular weight PEGs optionally containing one or more glycols. When using a hydrophilic component as the vehicle (i.e., the component used in the greatest amount, which can include one or more hydrophilic compounds), it should be preferably selected to maintain viscosity and melt temperature characteristics similar to those stated above for the anhydrous or nearly anhydrous formulations using a hydrophobic vehicle.
   Similarly the viscosity can be enhanced by either incorporating a crystalline or semicrystalline hydrophilic compound such as a PEG of sufficient molecular weight, addition of an insoluble filler/thixotrope, or by addition of a'polymeric thickener. Polymeric thickeners may be linear, branched, or slightly crosslinked. It is important for comfort that the formulations are relatively soft and that they spread easily to allow easy application, especially in the anterior nares or over a wound, rash, or infected area. For this reason, a particularly preferred vehicle is based on a blend of a liquid or semi-solid PEG (PEG 400-1000) with a more crystalline PEG (PEG 1000-2000). Particularly preferred is a blend of PEG 400 with PEG 1450 in a ratio of 4:1.
   In certain preferred embodiments of the present invention, the, compositions are in the form of an ointment or cream. That is, the compositions are in the form of a relatively viscous state such that they are suitable for application to nasal passageways.
(4) Neat Compositions: The antiseptic compositions of the present invention also may be delivered to the treatment site in a neat form or in a volatile solvent that rapidly evaporates to leave behind a neat composition. Such compositions may be solid, semi-solid or liquid. In the case where the compositions are solid, the antiseptic and/or the enhancer and/or the surfactant may optionally be microencapsulated to either sustain the delivery or facilitate manufacturing a powder which is easily delivered. Altematively, the composition'can be micronized into a fine powder without the addition of other components or it may optionally contain fillers and other ingredients that facilitate powder manufacture. Suitable powders include but are not limited to calcium carbonate, calcium phosphate, various sugars, starches, cellulose derivatives, gelatin, and polymers such as polyethylene glycols.

When hydrophobic antiseptics are used, a method for micronizing a hydrophobic agent may be used wherein the hydrophobic agent is dissolved in an effective amount of a first solvent that is free of polymer such as the method described in U.S. Patent No. 6,746,635. The hydrophobic agent and the solvent form a mixture having a continuous phase. A second solvent and then an aqueous solution are introduced into the mixture. The introduction of the aqueous solution causes precipitation of the hydrophobic agent and produces a composition of micronized hydrophobic agent having an average particle size of 1 micron or less. The particle size for use in delivery to the nose or other tissue may be significantly larger to direct delivery to the proper site. For example, to deliver the antiseptic powder to the nose, nasal cavities, and/or throat without passing into the lungs, larger particles may be required.

Bioadhesive polymers optionally may be added to the neat compositions as well as the other physical forms. Numerous suitable bioadhesive polymers are discussed in WO 93/21906. Representative bioadhesive polymers of particular interest include bioerodible hydrogels described by H. S. Sawhney, C. P. Pathak and J. A. Hubell in Macromolecules, 1993, 26:581-587, polyhyaluronic acids, casein, gelatin, glutin, polyanhydrides, polyacrylic acid, alginate, chitosan, poly(methyl methacrylates), poly(ethyl methacrylates), poly butylmethacrylate), poly(isobutylmethacrylate), poly(hexlmethacrylate), poly(isodecl methacrylate), poly(lauryl methacrylate), poly(phenyl methacrylate), poly(methyl acrylate), poly(isopropyl acrylate), poly(isobutyl acrylate), and poly(octadecl acrylate). Preferred polymers are polyacrylic acid (e.g., Carbomer) and poly(fumaric-co-sebacic)acid. Other bioadhesive and bioerodible polymers are described in U.S. Patent No. 6,746,635. Particularly preferred are slightly crosslinked polyacrylic acids such as those sold under the CARBOPOL brand by BF Goodrich.

The antimicrobial compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

The neat antiseptic compositions according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioXide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch. Those of skill in the art can readily determine the various parameters and conditions for producing aerosols without resort to undue experimentation. Inhaled , medications are preferred in some embodiments because of the direct delivery to the lung. Several types of metered dose inhalers are regularly used for administration by inhalation. These types of devices include metered dose inhalers (MDI), breath-actuated MDI, dry powder inhaler (DPI), spacer/holding chambers in combination with MDI, and nebulizers. Techniques for preparing aerosol delivery systems are well known to those of skill in the art. Generally, such systems should utilize components which will not significantly impair the biological properties of the agent (see, for example, Sciarra and Cutie, "Aerosols," in Remington's Pharmaceutical Sciences, 18the edition, 1990, pp. 1694-1712).

The compounds may also be formulated in rectal or vaginal compositions such as suppositories or retention enemas, e.g., containing conventional suppository bases such as cocoa butter or other glycerides.

### Viscosity

Certain preferred compositions of the present invention have a viscosity of at least 500 Centipoise (cps) for ease of application topically. Preferably, compositions of this invention have a viscosity of at least 1,000 cps, even more preferably at least 10,000 cps, even more preferably at least 20,000 cps, even more preferably at least 50,000 cps, even more preferably at least 75,000 cps, even more preferably at least 100,000 cps, and even more preferably at least 250,000 cps (and even as high as about 500,000 cps, 1,000,000 cps, or more). The viscosity can be measured as described below in the Viscosity Test. Preferred formulations have high viscosity even after application to mammalian tissue at 32-37°C. because certain optional ingredients, such as enhancers, hydrophilic compounds, hydrophobic compounds, and the like, may affect the viscosity (either positively or negatively), the measured viscosity is that of the final composition.

Lower viscosity compositions can be used, however, in certain applications, such as for the treatment of middle ear infection and chronic sinusitis. For example, afflictions of the middle ear (e.g., otitis media or infection of the middle ear) may be treated with compositions of the present invention having a viscosity lower than 1000 cps more readily by administration through the the outer ear or through the nose and into the Eustachian tubes. The viscosity is measured by the Viscosity Test described herein. Preferred compositions meet the above viscosity limitations even when warmed to 32°C. Most preferred compositions meet the above viscosity limitations even when warmed to 35°C.

### Delivery Methods and Devices

Antimicrobial compositions of the present invention can be provided to a medical professional in a single composite formulation or in multiple parts. For example, a composition can be provided in two parts (e.g., in two separate containers or two separate compartments of the same container), one part containing the antiseptic component and one part containing the enhancer. Other components of the composition can be combined with either one of the two parts. Alternatively, the other componenets can be included in a third part.

Topical antimicrobial treatment regimens according to the practice of this invention include applying a safe and effective amount of the compositions described herein directly to the infected or at-risk skin, wound, or mucous membrane; particularly, the nasal nares and passages that are particularly susceptible to microbial contamination. The dose and frequency of application will depend on many factors including the condition to be treated, the concentration of antiseptic and optional enhancer, the microbe to be killed, etc. Typically, the compositions will be delivered in dosages of at least 10 mg per cm² of tissue, preferably at least 20 mg per cm² of tissue, more preferably at least 30 mg per cm² of tissue, and most preferably at least 50 mg per cm² of tissue for most applications. Application can be made once, or several (e.g., 2-4) times daily for one or more days. Typically, the composition is applied 1 or 2 times/day for 1-7 days. For example, decolonization of the anterior nares may require a dose of 0.25 gram (g) per nares applied 1-3 times per day for 1-5 days. Treatment of impetigo may require about 0.5 g/15 cm² applied 1-3 times/day for 3-10 days.

Compositions of the present invention can be delivered using a variety or techniques. Typically, the compositions are delivered to the skin and/or mucosal tissue in a manner that allows them to penetrate into the skin and/or mucosal tissue, as opposed to through the tissue into the blood stream. This concentrates the compositions locally at the site in need of treatment. This delivery can be accomplished by spraying, dipping, wiping, dropping, pouring, toweling, inhaling, or the like, onto the area to be treated.

In the methods of the present invention, the antiseptic compositions may be provided as a formulation suitable for delivery to mammalian tissue (e.g., skin and/or mucosal surfaces). Suitable formulations can include, but are not limited to, creams, gels, foams, ointments, lotions, balms, waxes, salves, solutions, suspensions, dispersions, water in oil or oil in water emulsions, microemulsions, pastes, powders, oils, lozenges, boluses, and sprays, and the like.

The compositions may be sprayed from a pressurized container. The pressure may be supplied by an external means such as squeezing the container, through the use of a mechanical pump, or with the use of a propellant. Suitable propellants include chlorofluorocarbons (CFCs), hydrochlorofluorocarbons (HCFCs), hydrofluorocarbons (HFCs), hydrofluoroethers (HFEs), perfluorinated alkanes, and (C1-CS) alkanes as well as nitrous oxide and dimethyl ether.

If delivered as a foam, the composition may be dispensed from an aerating dispenser such as the F2 Finger Pump Foamer available from Air Spray International Pompano Beach, FL. Alternatively, the foam may be generated using a suitable propellant such as those described above.

For very high viscosity formulations the composition may be delivered in essentially a solid dosage form by placing the composition in or on the tissue to be treated. For example, a small suppository type delivery could be placed into the anterior nares for eradication of *staphylococcus sp.*

Various other modes of administration can be used as well known to one of skill in the art depending on the desired location for contact of the antimicrobial compositions of the present invention. For example, afflictions of the middle ear (e.g., otitis media or infection of the middle ear) may be treated with compositions of the present invention by administration through the nose and into the Eustachian tubes or they can be instilled directly into the middle ear through the tympanic membrane. The formulations may traverse the tympanic membrane with the aid of a syringe or do so by diffusion. Penetration enhancers may be used to enhance diffusion across the tympanic membrane.

For application to skin or mucosal tissue, for example, the compositions may be applied directly to the tissue from a collapsible container such as a flexible tube, blow/fill/seal container, pouch, capsule, etc. In this embodiment, the primary container itself is used to dispense the composition directly onto the tissue or it can be used to dispense the composition onto a separate applicator. For example, for delivery to the nose or other topical tissue, the composition could be dispensed directly from a tube and spread by a number of means including squeezing the outside of the nose together repeatedly, wiping with the tip of the tube or with a separate' device such as a spatula, cotton, rayon, or other natural or synthetic based fiber swab.

Other application devices may also be suitable including applicators with foam tips, brushes, and the like. Importantly, the applicator must be able to deliver the requisite amount of composition to the tissue. Therefore, in most instances applicator devices such as webs and swabs are coated on the applicator web at greater than 50% by weight of the dry web and preferably in excess of 100% by weight of the dry web. (On a swab this would include the weight only of the web and not the applicator stick.) The collapsible containers may be made in a number of single layer, laminate, or coexturded constructions. Materials of construction may include polyolefins such as low, medium or high density polyethylene including low and linear low density polyethylene, polypropylene, as well as copolymers of ethylene and/or propylene with other polar or non-polar comonomers; polyamides such as nylons, polyesters such as polyethylene terephalate, polybutyleneterephalate, polyethylenenaphthalate; polyurethanes, polyacrylates, and the like. In some constructions it may be desirable to include a barrier material to prevent evaporation of one or more components of the formulation. Suitable barrier materials include polyesters (e.g., polyethylene terephthalate, polyethylene naphthalate and polybutylene terephalate and the like), fluorinated layers such as polytetrafluoroethylene (PTFE, e.g., TEFLON), polyamides (e.g., nylon), chlorotriflouroethylene (ACLAR), polyvinylidene fluoride, as well as copolymers of perflourinated monomers with partially fluorinated monomers such as copolymers of tetraflouroethylene/hexafluoropropylene/vinylidene fluoride (THV Fluorothermoplastic from Dyneon Company), polyvinylchloride, polyvinylidene chloride (PVDC, e.g., SARAN HB), ethylene vinyl alcohol (EVOH), polyolefins (e.g., polyethylene, high density polyethylene, polypropylene, and combinations thereof). Oriented and biaxially oriented polymers may be particularly preferred.

Particularly preferred barrier constructions include metallic foil barriers such as aluminum foil laminates, HDPE, PET, PETG, PEN laminates of polyester and polyolefin (in particular PET/HDPE or HDPE/PET/HDPE), laminates of PET and EVOH, biaxially oriented nylon, PVDC, Nylon/EVOH/Nylon (OXYSHIELD OUB-R), chlorotrifluoroethylene and laminates thereof, ceramic layer including silicon oxide (SiOₓ where x = 0.5-2 and preferably 1-2) coated thermoplastics, and ceramic coated PET (CERAMIS available from CCL Container/Tube Division, Oak Ridge, NJ).

An antimicrobial composition may be applied to a mucosal surface with the use of a delivery device such as cervical caps, diaphragms and solid matrices such as tampons, cotton sponges, cotton swabs, foam sponges, and suppositories. Accordingly, compositions of the present invention can also be incorporated in (e.g., delivered from) cloth, sponges, paper products (e.g., paper towels, towelletes, and wipes), tampons_{;} undercast padding, and dental floss, for example.

In some embodiments, an applicator may be used to place the device and/or antimicrobial composition in the proper location, for example, on the mucosal surface of a vagina, nasal cavity, rectum, or the like. Examples of such applicators include, for example, cardboard or plastic tube applicators commonly used for inserting tampons or suppositories. The compositions of the present invention can be delivered from various substrates for delivery to the tissue. For example, the compositions can be delivered from a wipe or pad which when contacted to tissue will deliver at least a portion of the composition to the tissue. For application to nasal cavities the compositions may be provided by a non-woven swab such as a "Q-tip" brand cotton swab, into a foam tip applicator, and the like. The substrate may be used to deliver the composition essentially instantaneously or may be left in contact with the tissue. For example, a substrate in a tubular form could be delivered to the anterior nares using a suitable applicator and left in the anterior nares. The annular nature of the device is designed to allow delivery of the active while allowing the patient to freely breath through the nose.

Also, compositions of the present invention can be coated onto medical devices that contact skin, mucous membranes, wounds, etc. Example of such devices include catheters such as urinary tract catheters and vascular access catheters.

Objects and advantages of this invention are further illustrated by the following examples, but the particular materials and amounts thereof recited in these examples, as well as other conditions and details, should not be construed to unduly limit this invention.

### TEST PROTOCOLS

### KILLING MICROBES ON TISSUE

Many of the compositions of the present invention are intended to kill microorganisms on mammalian tissue such as skin and mucosal tissue. The extent of kill can be determined in the following manner. Subjects are identified who are naturally colonized with the microorganism of interest. This is preferred over methods where the tissue is artificially colonized with non-resident flora. For example, subjects may be identified whom are colonized with staphylococcus aureus (SA) in the anterior nares by swabbing the anterior nares and culturing the swab. This is normally repeated at least one additional time to ensure the subject is a "chronic carrier", i.e. one who carries the organism all or most of the time. A swab may also be taken several days prior to treatment to increase the probability that the subject is, in fact, a carrier. The subject is then treated with the indicated composition in a dose and at a frequency stated. The anterior nares once again are swabbed to determine if the bacteria has been reduced or eradicated (decolonized). Preferred formulations eradicate the SA in less than 5 days, preferably in less than 72 hours, more preferably in less than 48 hours, and most preferably in 24 hours or less. On skin the procedure is similar except that a control site distinct from the treatment site may be selected on the treatment day. In this case, a log reduction may be determined. The procedure on skin is described in Federal Register, 21 CFR Parts 333 and 369, Tentative Final Monograph for Healthcare Antiseptic Drug Products; Proposed Rule, 1994 (scrub cup method). When performing this method on skin the antiseptic compositions are generally allowed to remain in contact with the skin for at least 6 hours under a suitable dressing such as Tegaderm (3M Company) to check for antimicrobial activity. Preferred formulations show at least 1 log reduction and preferably at least 1.5 log reduction in 6 hours on a dry skin site (e.g. the abdomen).

### ANTIMICROBIAL EFFICACY TEST

This method tries to mimic the actual use conditions for many topical antiseptics. In most cases a topical antiseptic is applied to the area, optionally with some rubbing, and allowed to remain in contact and kill any microorganisms present in an essentially static state. In this assay, a composition is spread onto a film to form a uniform coating 10 mil (250 micron) thick, a suspension of bacteria are directly inoculated onto the surface of the composition, after a defined period of time, the inoculated disk is placed in a neutralizing broth, and at least a portion of this is diluted and plated to enumerate the surviving bacterial. It should be noted that just as in the in-vivo condition, this in-vitro method takes into account the ability of the formulation to be wet by tissue or the bacteria/bacterial suspension wetting. In certain compositions the bacterial suspension will wet the composition very well and spread. With other compositions the bacterial suspension may remain as discrete droplets. This is expected to simulate in-vivo performance in wetting tissue and bacterial biofilms. Since preferred compositions of the present invention are ointments this works very well. For less viscous compositions a compatible thickening agent should be incorporated to achieve a viscosity of at least 20,000 cps and preferably at least 50,000 cps.

For all antiseptics used in this assay an initial experiment was conducted to confirm that the neutralization broth was effective at neutralizing the antiseptic while not damaging the microorganisms. In general, to confirm neutralization, 100 uL of inoculum (target organism concentration of 10-100 CFU/mL) was added to 20 mL of warmed (36°C) neutralizer broth, vortexed, and a sample disk with ointment was dropped into the broth (time zero, t0) and the tube mixed vigorously. This was done using a vortex mixer for the 20 mL samples and by hand shaking for the 100 mL samples. 1 mL aliquots in duplicate were pour plated at three time points: 1) immediately (<1 minute), 2) at 30 minutes, and 3) at 60 minutes post-inoculation (all at room temperature). Plating was done using tryptic soy agar (TSA). Plates were incubated at 36°C for up to 48 hours. Plates were enumerated and CFU/mL calculated. The data was converted to log10 CFU/mL. Both test samples and a numbers control were run. The numbers control consisted of 100 µL of inoculum added to 20 mL PBW (phosphate buffered water, PBW) to yield an organism concentration of 10-100 CFU/mL. The PBW was prepared as follows: A stock solution was prepared by dissolving 34 g potassium dihydrogenphosphate in 500 mL deionized water. This was adjusted to pH 7.2 using 10N sodium hydroxide and then diluted with deionized water to make exactly 1 liter. The stock solution was filter sterilized and dispensed into a sterile bottle and refrigerated. The PBW was prepared by adding 1.25 mL stock solution to 1 liter deionized water and steam sterilized at 121 °C for 25 minutes. After sterilization, the solution was mixed by swirling to ensure uniformity. A toxicity control was also run by adding 100 µL of inoculum to 20 mL neutralizer broth to yield an organism concentration of 10-100 CFU/mL.

Neutralizer Effectiveness: If the 10g 10 CFU/mL of the test sample is not more than 0.3 log less than the corresponding Numbers Control, the neutralization will be considered effective.

Neutralizer Toxicity: If the Toxicity Control (TC) is not more than 0.3 log less than the corresponding Numbers Control sample, the sampling solution will be considered non-toxic.

### TEST ORGANISMS FOR ANTIMICROBIAL EFFICACY TEST

The test organism for this assay were methicillin resistant *Staphylococcus aureus,* ATCC 33953 and *E. coli,* ATCC 11229. The initial suspension was prepared by suspending bacterial colonies from overnight growth plates in phosphate-buffered water (PBW). A 0.5 McFarland turbidity standard was used to obtain a cell density of approximately 1.0 x 10⁸ CFU/mL.

### TEST MATERIALS FOR ANTIMICROBIAL EFFICACY TEST

The samples for this assay were spread at room temperature to a uniform thickness of 10 mil (250 µm) using a laboratory knife coater onto a 100 µm thick biaxially oriented clean and 70% isopropanol sanitized polyesterterephthalate (PET) film. These coated samples were placed in sterile petridishes and sealed with Parafilm to prevent evaporation and preserve cleanliness. Bubbles in the formulation were minimized as much as possible. Spread samples containing any volatile solvents such as water were used within 24 hrs of spreading. Test samples were cut from the same PET coated films using a 70% isopropyl alcohol (IPA) disinfected 23 mm die, as described in the next section. The sample disks were stored in sterile Petri dishes until testing.

Neutralizing Broth: The DE broth was Dey Eagle broth purchased as a solid and reconstituted according to directions from Difco Laboratoris, Detroit Michigan. The DE broth was used as a neutralizing broth for the examples containing lauric acid and tea tree oil. For the hydrogen peroxide containing examples, bovine liver catalase was added (purchased from Sigma Aldrich, Milwaukee, WI, having an activity of 47,400 units/ml). 20 µl was added to 20ml of the DE broth.

### INOCULUM PREPARATION FOR ANTIMICROBIAL EFFICACY TEST

The inoculum was serially diluted with phosphate buffered water (PBW) 10,000 fold (10⁻⁴) to achieve a concentration of 1-5 x 10⁴ CFU/mL. The inoculum suspension was enumerated at the beginning and end of the test period. The final count was within 0.1 10g/mL of the initial count. Each disk was inoculated with between 10^{6.5} and 10^{7.5} bacteria.

### MEASUREMENT OF ANTIMICROBIAL ACTIVITY:

After first confirming neutralization, samples were tested for antimicrobial activity using an *in vitro* model that attempts to simulate in-use conditions. Using aseptic technique and steam sterilized materials (except for the ointments), 23 mm disks of each formulation were cut using a 70% IPA-disinfected 23 mm die. Two bacteria were tested: *Staphylococcus aureus* (MRSA 33953) and *E. coli* ATCC 11229. Each inoculum was prepared by suspending bacterial colonies from overnight growth plates in phosphate-buffered water (PBW). A 0.5 McFarland turbidity standard was used to obtain a cell density of approximately 1.0 x 10⁸ CFU/mL. 50 µL of the inoculum was rapidly spotted on the surface of the test ointment (in 8-12 tiny droplets). After the last drop was applied the bacteria were allowed to remain in contact with the ointment for the specified period of time (e.g. 2.5 and 10 minutes). At the end of the exposure time (time bacteria are in contact with the composition) the inoculated disk was dropped into warm (36'C) Neutralizer Broth (20 mL) and mixed vigorously (vortexed using a VWR Vortex Genie 2) for 2 minutes for DE. Two one-hundred fold dilutions were prepared in Neutralizer Broth, and the bacteria enumerated using the pour plate. Plates were incubated at 36 °C for up to 48 hours. Colony Forming Units (CFUs) were counted.

The CFUs for each plates were multiplied by the dilution factor to arrive at CFU/mL, and converted to log10 CFU/sample. Log10CFU/sample of duplicate tests were averaged and the log10 reduction was calculated. Log reductions were calculated by subtracting the log 10 bacterial recovery of the test materials from the log 10 bacterial recovery of the control (100 µL of inoculum in 20 mL warm D/E neutralizing broth).

The compositions of the present invention were analyzed for their ability to kill MRSA and E.coli at 2.5 and 10 minutes. By comparison Bactroban Nasal ointment in this assay showed essentially no kill of this strain of MRSA at 2.5min. (The log reduction values were 0.030 and -0.040.) In fact, Bactroban Nasal showed essentially no kill after contact for 2 hours. It is a significant advantage that the compositions of the present invention are able to kill microorganisms rapidly. Preferred compositions achieve a at least a 1.5 log reduction in 10 minutes, more preferably at least a 2 log reduction in 10 minutes, and most preferably at least a 3 log reduction in 10 minutes. Particularly preferred compositions of the present invention achieve at least a 1.5 log reduction in 2.5 minutes, more preferably at least a 2 log reduction in 2.5 minutes, and most preferably at least a 3 log reduction in 2.5 minutes for at least one of the two test organisms. Most preferred formulations achieve these log reduction values for both test organisms.

### VISCOSITY TEST

For selected Examples viscosity was measured at approximately 22°C at ambient pressure using a Brookfield LVDV-I⁺ viscometer equipped with a model D Brookfield heliopath and LV spindles. The spindle and speed was chosen for each particular sample such that the viscometer was operating in the middle of its range. All samples were allowed to equilibrate at approximately 22°C for 24 hours prior to measurement. Preferably the viscosity is taken at the lowest speed possible while staying within 20-80% of the viscometer range and more preferably between 30-70% of the range. In all cases the sample size and container geometry was chosen to ensure that there were no wall effects. By "wall effects" sit is meant the viscosity value is not affected by the container and is essentially equivalent to the viscosity taken in an infinitely large container. For this reason lower viscosity samples required a larger sample size to accommodate the larger spindles.

### EXAMPLES

Objects and advantages of this invention are further illustrated by the following examples, but the particular materials and amounts thereof recited in these examples, as well as other conditions and details, should not be construed to unduly limit this invention. Table 1 Glossary of Components

| Acronym | Trade name | Description | Source | Address |
|---|---|---|---|---|
| | 2-phenoxyethanol | 2-phenoxyethanol | Aldrich | Milwaukee, WI |
| DOSS | Aerosol OT-75 | docusate sodium | American Cyanmid | W. Patterson, NJ |
| | Benzoic acid | benzoic acid | MallinckrodtBa ker | Paris, KY |
| | Carbowax 400 | Polyethyleneglycol 400 | DOW/Union Carbide | Danbury, CT |
| | Carowax 1450 | Higher MW PEG, e.g 1450 | DOW/Union Carbide | Danbury, CT |
| | Cerasynt GMS | glyceryl stearate | ISP | Lombard, IL |
| DOSS | Complemix | docusate sodium USP | ICI Americas | Wilmington, DE |
| | Glycerin (glycerol) | glycerin (glycerol) | Aldrich | Milwaukee, WI |
| | Hipure 88 | lactic acid (88%) | Purac America | Lincolnshire, IL |
| H₂0₂ | hydrogen peroxide 30.6% | hydrogen peroxide 30.6% | Aldrich Chemical | Milwaukee, WI |
| | Lauric acid | Lauric acid | Aldrich Chemical | Milwaukee, WI |
| | Mineral oil | Mineral oil USP | Paddock Labs | Minneapolis, MN |
| | Pluronic P-65 | nonionic difunctional block copolymer | BASF | Mount Olive, NJ |
| | Polawax | emulsifying wax, cetearyl alcohol + ceteareth 20 | Croda | Parsippany, NJ |
| | propylene glycol | 1,2 propanediol | JT Baker | Phillipsburg, NJ |
| | Snow White | White Petrolatum USP | Penreco | Karns City, PA |
| | Tea Tree Oil | Melaleuca | International Sourcing | Upper Saddle River, NJ |

### PREPARATION OF EXAMPLES:

Samples of 250 grams were prepared according to the procedures listed below. The samples were tested according to the Antimicrobial Efficacy test against both MRSA and E.coli at 2.5 minutes and 10 minutes.

### Control Examples C1 & C2

Control compositions of 250 grams each, containing no antimicrobial agents, were prepared using the components shown in table 2a for each example. Carbowax 1450 PEG was heated in an oven until melted in a first glass container. In a second glass container Glycerin, Carbowax 400 and Aerosol OT-75 were also heated to 70 °C. Contents of the second container were added to the first container, swirled by hand.to mix and reheated to 70 °C. The composition was removed from the oven and allowed to cool to at least approximately 40°C, while mixing on a roller.

### Example 1 (Not according to the invention)

An antimicrobial composition of 250 grams was prepared using the components shown in table 2a. Carbowax 1450 PEG was melted in an oven at approximately 70 °C in a glass container. Glycerin and Carbowax 400 were added to the container and swirled by hand to mix and then heated again to 70 °C. The remaining components (lauric acid, lactic acid, Complemix DOSS) were added to the container and mixed using a high shear rotor/stator Silverson homogenizer on high speed for 1 minute. The composition was allowed to cool on rollers to approximately 40°C then transferred into jars, and sealed.

### Examples 2-3 (Not according to the invention)

Antimicrobial compositions of 250 grams were prepared using the components shown in table 2a. Tea tree oil and Complemix DOSS were added to glycerin in a glass container and heated to 70°C in an oven. Carbowax 400 and Carbowax 1450 were added to the beaker, swirled by hand to mix and reheated to 70°C in the oven. The composition was removed from the oven allowed to cool to approximately 40°C, while mixing on rollers, then transferred into jars and sealed.

Examples 1-3 show that increasing the concentrations of Tea Tree oil or addition of an anionic surfactant improve the antimicrobial efficacy"to achieve complete kill in 10 minutes against *E. coli* and near complete kill against MRSA in only 2.5 minutes.

### Example 4 (Not according to the invention

An antimicrobial composition of 250 grams was prepared.in the same manner as examples 2-3, using the components shown in table 2a, except that lauric acid was used as the antimicrobial component instead of tea tree oil.

Example 4 shows that an alkyl carboxylic acid in a hydrophilic vehicle is capable of achieving complete kill against both MRSA and *E. coli* in 2.5 minutes or less.

### Example 5

An antimicrobial composition of 250 grams was prepared using the components shown in table 2b for each example. Petrolatum was added to a glass container and heated in an oven to approximately 70°C. Once the petrolatum was melted, lauric acid was added and allowed to dissolve. Complemix DOSS was then added to the container and mixed using a high shear rotor/stator Silverson homogenizer on high speed for 1 minute. Glycerin and lactic acid were then added and the composition was mixed again using a high shear rotor/stator Silverson homogenizer on high speed for 1 minute. Mixing was continued at low speed using a Gast overhead air mixer with radial flow impeller until just before the composition congealed at approximately 40°C. The composition was removed from the mixer, poured into jars, and sealed. Example 5, using a hydrophobic vehicle, akylcarboxylic acid, anionic surfactant, and a lactic acid enhancer, killed over 6 logs of MRSA in 2.5 minutes.

### Example 6 (Not according to the invention)

An antimicrobial composition of 250 grams was prepared using the components shown in table 2b for each example. Lauric acid, lactic acid and propylene glycol were added to a first glass container and heated to 70°C in an oven. Polawax and mineral oil were added to a second glass container and also heated to 70°C in an oven. Water was heated in the oven to 70°C in a third glass container. The water was then added to the second container and mixed using a high shear rotor/stator Silverson homogenizer on high speed for 1 minute. The contents of the first container were then added to the mixture of the second container and again mixed using a high shear rotor/stator Silverson homogenizer on high speed for 1 minute. The composition was allowed to cool on rollers to approximately 40°C.

The oil in water composition of Example 6, which comprised polyethoxylated surfactants (Polawax), appears to have inactivated the lauric acid.

### Examples 7-8

Antimicrobial compositions of 250 grams were prepared using the components shown in table 2b for each example. Petrolatum, glyceryl stearate and benzoic acid were added to a glass container and heated in an oven to approximately 70°C. Water was added to begin cooling of the mixture. Additionally, 2-phenoxyethanol was added to the container and once the temperature was below 40°C. hydrogen peroxide was then added to the mixture as a 30% solution. Finally, the composition was mixed using a high shear rotor/stator Silverson homogenizer on high speed for 1 minute.

The higher efficacy of Example 8 illustrates the advantage of adding a carboxylic acid enhancer into peroxide containing compositions.

### Component Composition of Examples:

Table 2a and 2b shows the weight/weight % concentration of each component in each example composition, as well as the antimicrobial efficacy results.

| Table 2a | Example Numbers | | | | | |
|---|---|---|---|---|---|---|
| | C1 | C2 | 1 | 2 | 3 | 4 |
| Component | w/w % amount of components | | | | | |
| Tea tree oil | - | - | 2.00 | 3.00 | 5.00 | - |
| Lauric Acid' | - | - | - | - | - | 3.00 |
| Hipure 88 (lactic acid) | - | - | - | - | - | 1.00 |
| Carbowax 400 | 61.78 | 60.96 | 57.00 | 59.00 | 58.50 | 58.66 |
| Carowax 1450 | 16.75 | 16.53 | 16.00 | 16.50 | 16.25 | 15.89 |
| Glycerin | 21.47 | 21.18 | 20.00 | 20.50 | 20.25 | 20.45 |
| Complemix | - | - | - | 1.00 | - | 1.00 |
| Aerosol OT-75 (DOSS) | - | 1.33 | - | - | - | - |
| Pluronic P-65 | - | - | 5.00 | - | - | - |
| Total | 100.00 | 100.00 | 100.00 | 100.00 | 100.00 | 100.00 |
| Antimicrobial efficacy results: | | | | | | |
| 2.5 min MRSA test 1 | -0.8 | -0.2 | 0.1 | 6.6 | NT | 6.7* |
| 2.5 min MRSA test 2 | -0.8 | -0.3 | 0.1 | 5.9 | NT | 6.7* |
| Average | -0.8 | -0.3 | 0.1 | 6.3 | - | 6.7* |
| | | | | | | |
| 2.5 min E coli test 1 | -0.5 | 0.1 0.9 | 0.4 | NT | NT | 7.0* |
| 2.5 min E coli test 2 | -0.5 | 0.1 0.7 | -0.4 | NT | NT | 7.0* |
| Average | -0.5 | 0.5* | 0:0 | - | - | 7.0* |
| * Average of 2 sets of 2 | | | | | | |
| | | | | | | |
| 10 min MRSA test 1 | NT | NT | 0.6 | 8.1 | NT | NT |
| 10 min MRSA test 2 | NT | NT | 0.5 | 6.5 | NT | NT |
| Average | - | - | 0.6 | 7.3 | - | - |
| | | | | | | |
| 10 min E coli test 1 | NT | NT | 2.9 | 6.6* | 6.6* | NT |
| 10 min E coli test 2 | NT | NT | 0.6 | 6.6* | 6.6* | NT |
| Average | - | - | 1.8 | 6.6* | 6.6* | - |

| | | | | | | |
|---|---|---|---|---|---|---|
| * Complete kill. | | | | | | |

| Table 2b | Example Numbers | | | |
|---|---|---|---|---|
| | 5 | 6 | 7 | 8 |
| Component | w/w % amount of components | | | |
| 2-phenoxyethanol | - | - | 0.50 | 0.50 |
| Lauric Acid | 3.00 | 3.00 | - | - |
| H₂0₂ 30.6% in water | - | - | 3.27 | 3.27 |
| Hipure 88 (lactic acid) | 1.00 | 1.00 | - | - |
| Benzoic acid | - | - | - | 0.50 |
| Glycerin | 10.00 | - | - | - |
| Propylene glycol | - | 20.00 | - | - |
| Cerasynt GMS | - | - | 10.00 | 10.00 |
| Polawax | - | 10.00 | - | - |
| Mineral oil | - | 5.00 | - | - |
| Snow White Petrolatum | 85.00 | - | 58.00 | 57.50 |
| Complemix (DOSS) | 1.00 | - | 1.00 | 1.00 |
| Water | - | 61.00 | 27.73 | 27.73 |
| Total | 100.00 | 100.00 | 100.00 | 100.00 |
| Antimicrobial efficacy results: | | | | |
| 2.5 min MRSA test 1 | 5.7 | 0.6 | -0.1 | 3.8 |
| 2.5 min MRSA test 2 | 6.7 | 0.3 | 0.1 | 3.9 |
| Average | 6.2 | 0.5 | 0.0 | 3.8 |
| | | | | |
| 2.5 min E coli test 1 | 0.2 | -0.4 | 1.0 | 6.8* |
| 2.5 min E coli test 2 | 0.2 | -0.3 | 1.0 | 6.8* |
| Average | 0.2 | -0.3 | 1.0 | 6.8* |

| | | | | |
|---|---|---|---|---|
| * Complete Kill No testing performed at 10 minutes for MESA or E coli. | | | | |

### SUBJECT ACCEPTABILITY OF PLACEBO - FIRST PANEL EVALUATION

A panel of 10 normal healthy volunteers of either gender over 18 years of age evaluated a component composition without active antiseptic to determine acceptability and to develop evaluation methodology for future evaluation.

The compositions evaluated are shown in Table 3.

| Table 3 | | | | | | |
|---|---|---|---|---|---|---|
| Composition | Components (weight percent) | | | | | |
| | Lactic Acid USP | Glycerin USP | Docuate sodium USP (50%) | White petrolatum USP | PEG 400 NF | PEG 3350 NF |
| W | 1.00 | 10.00 | 2.00 | 87.00 | 0.00 | 0.00 |
| X | 1.00 | 20.00 | 2.00 | 0.00 | 59.00 | 18.00 |

### Test Procedure

A dose was 0.5 mL of Composition W or X applied using a preloaded 1 mL plastic syringe. The volunteers applied the first dose after viewing a demonstration of the technique. The volunteers applied a second and third dose during Day 1.

One-half of the volunteers (5) were dosed with Composition W and one-half of the volunteers were dosed with Composition X on Day 1 and given a Rhinoscopic Examination of Nares before and after application on Day 1 and after 24 hours on Day 2. On Day 8 those volunteers dosed with Composition W on Day 1 received Composition X and those dosed with Composition X on Day 1 received Composition W. They were given a Rhinoscopic Examination of Nares before and after application on Day 8 and after 24 hours on Day 9.

Volunteers completed a questionnaire on Day 1 and on Day 9.

### Results:

All 10 volunteers successfully completed both periods of the study. Descriptive analysis was provided for each categorical variable in the study.

Composition W was preferred by 10/10 of the volunteers. Five of ten volunteers could not complete all three application of Composition X. They cited stinging, burning and runny noses as primary reasons. Composition X caused more rhinorrhea than Composition W. Volunteers using Composition X felt they could use the ointment for a shorter period of time than with Composition W. Composition W could be felt to remain in the nasal vestibule longer (mean 218 minutes) than Composition X (mean 145 minutes).

### SUBJECT ACCEPTABILITY OF PLACEBO - SECOND PANEL EVALUATION

A second panel evaluation was done to determine acceptability of essentially anhydrous ointments based hydrophobic vehicles containing lactic acid or mandelic acid. The criteria for the panel were the same as for the first panel. The compositions evaluated are given in Table 4.

| Table 4 | | | | | |
|---|---|---|---|---|---|
| Composition | Components (weight percent) | | | | |
| | Lactic Acid USP | Mandelic Acid | DOSS USP (50%) | Glycerin USP | White petrolatum USP |
| Y | 1.00 | 0.00 | 2.00 | 10.00 | 87.00 |
| Z (emulsion) | 0.00 | 1.00 | 2.00 | 10.00 | 87.00 |

The test procedure was the same as that used for the first panel except a cotton swab was used to apply the composition rather than a tube.

### Results:

Both ointments were acceptable with minimal, if any, side effects. The preference for the two ointments was fairly equally divided. Four of ten volunteers expressed a slight preference for the mandelic acid composition, three of ten volunteers expressed a slight preference for the lactic acid composition, and three of ten volunteers noticed no difference between the compositions.

Each volunteer applied 0.5 mL of composition; however, approximately 0.1 gram was routinely left on the swab. Therefore the dose was about 0.2 mL per nares. The time that the ointments remained in the volunteers' noses varied between volunteers, but there were indications that the ointment remained in place up to 24 hours. Two volunteers reported that the ointment appeared to accumulate from application to application.

The feel of the ointment in the nose and smell were the most noticed characteristics of both ointments, but the characteristics were all in the acceptable range.

### VISCOSITY TEST RESULTS

The viscosity of select examples are shown in Table 5. These were tested at approximately 22 °C (72°F) in accordance with the Viscosity Test.

| Table 5 | |
|---|---|
| Example No. | Viscosity cP x 1000 |
| C1 | 60 |
| C2 | 70 |
| 4 | 190 |
| 5 | 1360 |
| 6 | 196 |

## Claims

1. An antimicrobial composition comprising:
an antiseptic selected from the group consisting of C6-C 14 alkyl carboxylic acids, C6-C14 alkyl carboxylate ester carboxylic acids, C8-C22 mono- or polyunsaturated carboxylic acids, and natural oils;
an effective amount of an enhancer component which enhances the effectiveness of the antiseptic, wherein the enhancer component comprises an alpha-hydroxy acid, a beta-hydroxy acid, a chelating agent, a (C1-C4)alkyl carboxylic acid, a (C6-C12)aryl carboxylic acid, a (C6-C12)aralkyl carboxylic acid, a (C6-C16)alkaryl carboxylic acid, a phenolic compound, a (C1-C10)alkyl alcohol, an ether glycol, or combinations thereof;
a hydrophilic component selected from water, polyhydric alcohols having a molecular weight of less than 500, lower alkyl ethers, N-methylpyrrolidon, alkyl esters, C₁-C₁₀ monohydroxy alcohols, and combinations thereof; wherein the lower alkyl ethers and the alkyl esters have a sufficiently small number of carbon atoms to have a solubility in water of at least 7 wt.-% at 23°C, wherein the hydrophilic component is present in an amount of at least 1 wt.-%; and
a hydrophobic component which forms the greatest portion of the composition for killing or inactivating microorganisms on mammalian tissue.

2. The composition of claim 1, wherein the hydrophobic component is present in an amount of 50 wt.-% to 99 wt.-%.

3. The composition of claim 1, wherein the hydrophilic component comprises a glycol, a lower alcohol ether, a short chain ester, or combinations thereof, and wherein the hydrophilic component is soluble in water in an amount of at least 20 wt.-% at 23°C.

4. The composition of claim 1, wherein the hydrophobic component is an organic compound that is liquid, gelatinous, semisolid, or solid at 23°C and has a solubility in water of less than 5 wt.-% at 23°C.

5. The composition of claim 1, wherein the composition achieves at least 2 log reduction in test bacteria in 10 minutes according to the Antimicrobial Efficacy Test as disclosed in the description.

6. The composition of claim 1, wherein the composition achieves at least 4 log reduction in test bacteria in 10 minutes according to the Antimicrobial Efficacy Test as disclosed in the description.

7. The composition of claim 1, wherein the microorganisms comprise bacteria and the antimicrobial composition is used in an amount effective to kill one or more bacteria.

8. The composition of claim 7, wherein the bacteria comprise *Staphylococcus spp., Streptococcus spp., Escherichia spp., Enterocuccus spp.,* or *Pseudomonas spp.* and combinations thereof.

9. The composition of claim 7, wherein the bacteria comprise *Staphylococcus aureus, Staphylococcus epidermidis, Escherichia coli, Pseudomonas aeruginosa,* or *Streptococcus pyogenes* and combinations thereof.

10. The composition of claim 1, wherein the microorganisms comprise one or more viruses and the antimicrobial composition is used in an amount effective to inactivate one or more viruses.

11. The composition of claim 1, wherein the microorganisms comprise one or more fungi and the antimicrobial composition is used in an amount effective to kill one or more fungi.

12. The composition of claim 1, wherein the antiseptic is present in a concentration of at least 75% of the solubility limit of the antiseptic in the hydrophobic component.

13. The composition of claim 1, wherein the composition comprises a water-in-oil emulsion.

## Patentansprüche

1. Antimikrobielle Zusammensetzung, umfassend:
ein Antiseptikum, ausgewählt aus der Gruppe bestehend aus (C6-C14)-Alkylcarbonsäuren, (C6-C14)-Alkylcarboxylatestercarbonsäuren, einfach oder mehrfach ungesättigten (C8-C22)-Carbonsäuren und natürlichen Ölen;
eine wirksame Menge einer Verstärkerkomponente, welche die Wirksamkeit des Antiseptikums verstärkt, wobei die Verstärkerkomponente eine alpha-Hydroxysäure, eine beta-Hydroxysäure, einen Chelatbildner, eine (C1-C4)-Alkylcarbonsäure, eine (C6-C12)-Arylcarbonsäure, eine (C6-C12)-Aralkylcarbonsäure, eine (C6-C16)-Alkarylcarbonsäure, eine phenolische Verbindung, einen (C1-C10)-Alkylalkohol, ein Etherglykol oder Kombinationen davon umfasst;
eine hydrophile Komponente, ausgewählt aus Wasser, polyhydrischen Alkoholen mit einem Molekulargewicht von weniger als 500, Niederalkylethern, N-Methylpyrrolidon, Alkylestern, (C1-C10)-Monohydroxyalkoholen und Kombinationen davon; wobei die Niederalkylether und die Alkylester eine ausreichend kleine Anzahl von Kohlenstoffatomen aufweisen, um eine Löslichkeit in Wasser von wenigstens 7 Ges.-% bei 23°C aufzuweisen, wobei die hydrophile Komponente in einer Menge von wenigstens 1 Gew.-% vorhanden ist; und
eine hydrophobe Komponente, die den größten Teil der Zusammensetzung zum Abtöten oder Inaktivieren von Mikroorganismen auf Säugergewebe bildet.

2. Zusammensetzung gemäß Anspruch 1, wobei die hydrophobe Komponente in einer Menge von 50 Gew.-% bis 99 Gew.-% vorhanden ist.

3. Zusammensetzung gemäß Anspruch 1, wobei die hydrophile Komponente ein Glykol, einen Niederalkoholether, einen kurzkettigen Ester oder Kombinationen davon umfasst, und wobei die hydrophile Komponente in einer Menge von wenigstens 20 Gew.-% bei 23 °C in Wasser löslich ist.

4. Zusammensetzung gemäß Anspruch 1, wobei die hydrophobe Komponente eine organische Verbindung ist, die bei 23 °C flüssig, gallertartig, halbfest oder fest ist und eine Löslichkeit in Wasser von weniger als 5 Gew.-% bei 23 °C aufweist.

5. Zusammensetzung gemäß Anspruch 1, wobei die Zusammensetzung eine wenigstens 2 log-Verringerung von Testbakterien innerhalb von 10 Minuten gemäß dem in der Beschreibung offenbarten antimikrobiellen Wirksamkeitstest erzielt.

6. Zusammensetzung gemäß Anspruch 1, wobei die Zusammensetzung eine wenigstens 4 log-Verringerung von Testbakterien innerhalb von 10 Minuten gemäß dem in der Beschreibung offenbarten antimikrobiellen Wirksamkeitstest erzielt.

7. Zusammensetzung gemäß Anspruch 1, wobei die Mikroorganismen Bakterien umfassen und die antimikrobielle Zusammensetzung in einer zum Abtöten von einem oder mehreren Bakterien wirksamen Menge verwendet wird.

8. Zusammensetzung gemäß Anspruch 7, wobei die Bakterien *Staphylococcus spp., Streptococcus* spp., *Escherichia spp., Enterococcus spp.* und *Pseudomonas spp.* und Kombinationen davon umfassen.

9. Zusammensetzung gemäß Anspruch 7, wobei die Bakterien *Staphylococcus aureus, Staphylococcus epidermidis, Escherichia coli, Pseudomonas aeruginosa* und *Streptococcus pyogenes* und Kombinationen davon umfassen.

10. Zusammensetzung gemäß Anspruch 1, wobei die Mikroorganismen ein oder mehrere Viren umfassen und die antimikrobielle Zusammensetzung in einer zum Inaktivieren von einem oder mehreren Viren wirksamen Menge verwendet wird.

11. Zusammensetzung gemäß Anspruch 1, wobei die Mikroorganismen einen oder mehrere Pilze umfassen und die antimikrobielle Zusammensetzung in einer zum Abtöten von einem oder mehreren Pilzen wirksamen Menge verwendet wird.

12. Zusammensetzung gemäß Anspruch 1, wobei das Antiseptikum in einer Konzentration von wenigstens 75 % der Löslichkeitsgrenze des Antiseptikums in der hydrophoben Komponente vorhanden ist.

13. Zusammensetzung gemäß Anspruch 1, wobei die Zusammensetzung eine Wasser-in-Öl-Emulsion umfasst.

## Revendications

1. Composition antimicrobienne comprenant :
un antiseptique choisi dans le groupe constitué par les acides alkyl (en C₆-C₁₄) carboxyliques, les acides carboxyliques d'ester carboxylate d'alkyle en C₆-C₁₄, les acides carboxyliques mono- ou poly-insaturés en C₈-C₂₂, et les huiles naturelles ;
une quantité efficace d'un constituant améliorateur qui améliore l'efficacité de l'antiseptique, le constituant améliorateur comprenant un alpha-hydroxyacide, un bêta-hydroxyacide, un agent chélateur, un acide alkyl(en C₁-C₄) carboxylique, un acide aryl(en C₆-C₁₂) carboxylique, un acide aralkyl (en C₆-C₁₂) carboxylique, un acide alkaryl (en C₆-C₁₆) carboxylique, un composé phénolique, un alcool alkylique en C₁-C₁₀, un éther-glycol, ou leurs combinaisons ;
un constituant hydrophile choisi parmi l'eau, les polyols ayant une masse moléculaire inférieure à 500, les éthers d'alkyle inférieur, la N-méthylpyrrolidone, les esters alkyliques, les monohydroxyalcools en C₁-C₁₀, et leurs combinaisons ; dans lequel les éthers d'alkyle inférieur et les esters alkyliques ont un nombre d'atomes de carbone suffisamment faible pour avoir une solubilité dans l'eau d'au moins 7 % en poids à 23°C, dans lequel le constituant hydrophile est présent à raison d'au moins 1 % en poids ; et
un constituant hydrophobe qui forme la majeure partie de la composition, permettant de tuer ou d'inactiver les microorganismes sur un tissu mammalien.

2. Composition selon la revendication 1, dans laquelle le constituant hydrophobe est présent à raison de 50 % en poids à 99 % en poids.

3. Composition selon la revendication 1, dans laquelle le constituant hydrophile comprend un glycol, un éther d'alcool inférieur, un ester à chaîne courte, ou leurs combinaisons, et dans laquelle le constituant hydrophile est soluble dans l'eau à raison d'au moins 20 % en poids à 23°C.

4. Composition selon la revendication 1, dans laquelle le constituant hydrophobe est un composé organique qui est liquide, gélatineux, semi-solide ou solide à 23°C et qui possède une solubilité dans l'eau inférieure à 5 % en poids à 23°C.

5. Composition selon la revendication 1, dans laquelle la composition permet d'obtenir une réduction d'au moins 10² des bactéries testées en 10 minutes conformément à l'essai d'efficacité antimicrobienne décrit dans la description.

6. Composition selon la revendication 1, dans laquelle la composition permet d'obtenir une réduction d'au moins 10⁴ des bactéries testées en 10 minutes conformément à l'essai d'efficacité antimicrobienne décrit dans la description.

7. Composition selon la revendication 1, dans laquelle les microorganismes comprennent des bactéries et la composition antimicrobienne est utilisée en quantité efficace pour tuer une ou plusieurs bactéries.

8. Composition selon la revendication 7, dans laquelle les bactéries comprennent *Staphylococcus spp., Streptococcus spp., Escherichia spp., Enterococcus spp.* ou *Pseudomonas spp.,* et leurs combinaisons.

9. Composition selon la revendication 7, dans laquelle les bactéries comprennent *Staphylococcus aureus, Staphylococcus epidermidis, Escherichia* coli, *Pseudomonas aeruginosa* ou *Streptococcus pyogenes,* et leurs combinaisons.

10. Composition selon la revendication 1, dans laquelle les microorganismes comprennent un ou plusieurs virus et la composition antimicrobienne est utilisée en quantité efficace pour inactiver un ou plusieurs virus.

11. Composition selon la revendication 1, dans laquelle les microorganismes comprennent un ou plusieurs champignons et la composition antimicrobienne est utilisée en quantité efficace pour tuer un ou plusieurs champignons.

12. Composition selon la revendication 1, dans laquelle l'antiseptique est présent à une concentration d'au moins 75% de la limite de solubilité de l'antiseptique dans le constituant hydrophobe.

13. Composition selon la revendication 1, dans laquelle la composition comprend une émulsion eau dans l'huile.
